# EUROPEAN PATENT SPECIFICATION

(11) **EP 1 135 481 B1**
(45) Date of publication and mention of the grant of the patent: **25.02.2004**
(21) Application number: 99959296.7
(22) Date of filing: 22.11.1999
(51) Int. Cl.: C12N 15/11, A61K 47/48, A61K 48/00, A61P 35/00, A61P 37/00

(54) **NEW OLIGOMERIC CONJUGATES LIABLE TO TRANSFER BIOLOGICAL MOLECULES INTO CELLS**
NEUE OLIGOMERE KONJUGATE ZUM TRANSFER BIOLOGISCHER MOLEKÜLE IN ZELLEN
NOUVEAUX CONJUGUES OLIGOMERES APTES A TRANSFERER DES MOLECULES BIOLOGIQUES DANS DES CELLULES

(30) Priority: 02.12.1998 EP 98403015
(43) Date of publication of application: 26.09.2001
(73) Proprietor: I.D.M. IMMUNO-DESIGNED MOLECULES, 75011 Paris (FR)
(72) Inventor: MIDOUX, Patrick, F-45100 Orléans (FR); PICHON, Chantal, F-45640 Sandillon (FR); BELLO-ROUFAI, Mahajoub, 45072 Orléans Cedex 02 (FR); MONSIGNY, Michel, F-45590 Saint-Cyr-en-Val (FR)
(74) Representative: Grosset-Fournier, Chantal Catherine
(86) International application number: PCT/EP1999/008980
(87) International publication number: WO 2000/032764

(56) References cited:
- EP-A- 0 727 223
- WO-A-98/19710
- WO-A-98/22610
- US-A- 5 627 270
- GOTO T. ET AL: "A novel approach for gene medicine;synthetic poly-L- lysine /serine copolymer enhances bioactivity of antisense oligonucleotides." NUCLEOSIDES AND NUCLEOTIDES, (1997) 16/7-9 (1609-1615). REFS: 10 ISSN: 0732-8311 CODEN: NUNUD5, XP002108844 United States
- MIDOUX P ET AL: "Membrane permeabilization and efficient gene transfer by a peptide containing several histidines." BIOCONJUGATE CHEMISTRY, (1998 MAR-APR) 9 (2) 260-7. JOURNAL CODE: A1T. ISSN: 1043-1802., XP002108845 United States

## Description

The invention relates to new oligomeric conjugates liable to favor the transfer of biological molecules such as oligonucleotides, peptides and oligosides into cells.

The introduction of such molecules into cells is of great therapeutical interest.

Antisense oligonucleotides (ODN) and triplex forming oligonucleotides (TFO) are examples of attractive putative drugs in inhibiting or regulating gene expression in tumor cells and virus-infected cells.

Peptides from tumors and viruses are also attractive molecules in stimulating or elicitating a cell defense involving cytotoxic T lymphocytes against tumor and viruses after presentation by antigen presenting cells (APCs) such as dendritic cells, macrophages and B cells.

But to be effective these molecules must reach their target in the right intracellular compartments which are either the cytosol or the nucleus.

Until now, studies of the intracellular location of oligonucleotides point out that in the majority of cells, most of the oligonucleotides are confined inside vesicles once taken up and only a small amount succeeds in reaching their RNA and DNA targets in the cytosol and in the cell nucleus.

Because once in the cytosol, the oligonucleotides penetrate rapidly into the nucleus, the enhancement of the delivery of oligonucleotides into the cytosol upon cell uptake, is expected to increase their biological activity.

Oligonucleotide encapsulation into liposomes increases their delivery into the cytosol but efficiency is drastically reduced in the presence of serum.

Antigenic peptides can bind to MHC Class I molecules and be presented at the cell surface of APCs, upon their processing via proteasomes located in the cytosol.

Usually, peptides are taken up by APCs inside vesicles and delivered to lysosomes where they are either degraded or processed for an MHC Class II molecules presentation. Restricted MHC Class I molecules presentation by APCs requires that peptides must be introduced into the cytosol.

In French Patent n° 9613990 and PCT/FR97/02022, it has been shown that histidylated polylysine complexed with a nucleic acid is a system for cell transfection. The nucleic acid has a 10⁶ to 10⁸ of molecular weight. Polylysine is substituted at least 10% advantageously from 15% to 35% with molecules inducing membrane destabilization at acidic pH (mainly histidyl residues), and polylysine has a degree of polymerization of 15 to 900, particularly 200.

However, further studies have shown that the above-mentioned complex, which allows the transfection of cells by DNA, does not allow the transfer of an ODN (oligonucleotide).

The document WO 98/19710 relates to a method of constructing a synthetic polymer-based carrier vehicle for delivery of nucleic acid material to target cells in biological systems, said method comprising the sequential steps of (a) bringing the nucleic acid material into association with cationic polyelectrolyte polymer material to form by self-assembly therebetween a polyelectrolyte complex which provides a nucleic acid containing cationic polymer core for said carrier vehicle, and (b) reacting said polyelectrolyte complex with reactive hydrophilic polymer material so that the latter bonds to said complex and forms a hydrophilic coating that provides an outer protective steric shield and assists in stabilising the complex. The cationic polyelectrolyte polymer material is particularly composed of polyamine molecules and preferably of polylysine. This document also describes the synthesis of a polylysine partially substituted with histidine, in which the degree of substitution of the polylysine is comprised between 5% and 30%. The method described in this document allows the transfer of DNA, but it is not efficient for the transfer of oligonucleotides.

The document EP 0 727 223 relates to a poly-amino acidic oligonucleotide carrier comprising a poly-lysine:serine random copolymer. This document does not mention an histidylated polylysine oligomer.

The document Midoux et al. (Bioconjugate Chemistry, 1998, 9(2): 260-267) relates to a peptide that permeabilizes cell membrane at a slightly acidic pH. This peptide is analogous to the N-terminal segment of the HA-2 subunit of the influenza virus hemagglutinin, in which the amino acids in position 4, 8, 11, 15 and 19 have been replaced by histidyl residues and the amino acid in position 17 has been replaced by a leucyl residue. This peptide is not positively charged at a neutral pH and it does not form complexes with oligonucleotides. Thus, it is not efficient for the transfer of oligonucleotides.

One aim of the invention is to provide new positively charged oligomeric conjugates enabling the transmembrane passage of water soluble oligomers such as oligonucleotides, peptides and oligosides into the cytosol.

An other aim of the invention is to provide new oligomeric conjugates of substituted oligolysine liable to allow the transfer of oligonucleotides, peptides and oligosides into cells.

One advantage of the invention is that the formation of a complex between new oligomeric conjugates and oligoanions such as oligonucleotides, anionic peptides or anionic oligosides, is not required to allow their transfer into the cytosol and/or the cell nucleus.

Another advantage of the invention is that, although it is not excluded, the transfer of oligoanions such as oligonucleotides, anionic peptides, anionic oligosides (i.e. sulphated, phosphorylated, succinylated or sialytaded oligosides) or a mixture thereof, does not require the formation of electrostatic complexes with the new positively charged oligomeric conjugates.

Another aim of the invention is to provide an *in vitro, ex vivo* and *in vivo* transfer process.

Another aim of the invention is to provide defined oligmeric compounds in which the nature of the monomeric compounds can be different from each other.

This has been achieved through the invention.

The invention, in one of its most general definitions, concerns a positively charged oligomeric conjugate containing an oligomer with a polymerization degree (PD) from 5 to 50, preferably 10 to 40 and more preferably 20, formed from monomeric components having free NH₃⁺ in a number equal to or higher than 50 % of the polymerization degree,
said oligomer being as follows :
- the free NH₃⁺ of the above-mentioned components are substituted in a ratio of at least 50 %, advantageously from 60 % to 95 %, particularly 80 to 90 % (this ratio being determined by nuclear magnetic resonance), by protonable residues in a weak acid medium, leading in such weak acid medium to a destabilization of cellular membranes,
- the above-mentioned protonable residues possess in addition the following properties :
   → they contain a functional group enabling them to be linked to the above-mentioned oligomer,
   → they do not correspond to a recognition signal recognized by a cellular membrane receptor,
   → they can comprise at least one free NH₃⁺ group,
- the free NH₃⁺ of the above-mentioned monomers can be also substituted by an uncharged residue leading to a reduction of the number of positive charges in comparison to the same oligomeric conjugate, before substitution,
- molecules constituting a recognition signal recognized by a membrane cellular receptor may be present :
   → either by substitution of some of the free NH₃⁺ of the above-mentioned monomers,
   → either on some of the uncharged residues leading to a reduction of the number of charges,
   → either on some of the above-mentioned protonable residues leading to a destabilization of the cellular membranes,
   → or by substitution of the free NH₃⁺ (if it is present) of the above-mentioned protonable residues leading to a destabilization of the cellular membrane,
provided that :
1) the total number of the non substituted NH₃⁺ is of at least 50 % of the polymerization degree,
2) the number of monomers initially carrying free NH₃⁺ is substituted in a ratio of at least 50 % of the polymerization degree by residues leading to a destabilization of the cellular membrane.

As it will result from the following, the first proviso corresponds to m ≥ i/2 and the second proviso corresponds to u ≥ i/2.

Said oligomeric conjugate of the invention leads to the destabilization of the membranes and allows the transfer of the above-mentioned biomolecules in the cytosol and/or the nucleus of the cells.

According to a quite unexpected effect, the oligomeric conjugates of the invention allow the transfer of ODN into the cytosol and the nucleus, without allowing the transfection of cells by DNA.

The originality of the invention lies in the fact that the oligomer of the invention must be substituted to a level of more than 50 % with molecules inducing a membrane destabilization at acidic pH (amongst such molecules are histidyl residues) because oligomeric conjugates substituted at a level lower than 50 % are cytotoxic (see table 1, hereafter). Moreover, the toxicity of oligomeric conjugates substituted at a level lower than 50 % increases with the number of free NH₃⁺ of the unsubstituted lysyl residues.

For instance, for a oligomer of lysine of DP of 20 and carrying histidyl residues leading to the destabilization of the membranes, the above-said conditions can be expressed as follows :
- there must be at least 10 free NH₃⁺, which come from the α-NH₃⁺ and/or ε -NH₃⁺,
- there are at least 10 histidyl residues, and even up to 20 histidyl residues carried by the side chain of the lysine residues which means, in the latter case, that the minimum amount of free NH₃⁺ required, i.e. "at least" 10 free NH₃⁺ come only from the α-NH₃⁺ functions of the histidyl residues.

The destabilization of membranes means a modification of membranes which leads either to the increase of their permeability with respect to low molecular weight (and possibly high molecular weight) molecules in solution, or the fusion with another membrane.

The membrane permeability can be measured as follows :

Cells are incubated at 37°C for 30 min in DMEM medium without serum in the presence of 0.5 mg/ml fluorescein-labelled dextran (Mw 4000) and in the absence or in the presence of an oligomeric conjugate. Cells are then washed and incubated for 30 min at 37°C in culture medium containing 10% serum. Cells are fixed for 5 min in PBS containing 4 % paraformaldehyde and the cell fluorescence localization is analysed under a fluorescent confocal microscope.

The fusion of membrane can be measured as follows :

The fusion of membrane can be measured by using liposomes according to Struck et al., (Use of resonance energy transfer to membrane fusion. 1981 Biochemistry 20: 4093-4099).

Dioleoylphosphatidylcholine (DOPC) liposomes containing N-(7-nitrobenz-2-oxa-1,3-diazol-4-yl)-1,2-dihexadecanoyl-sn-glycero-3-phosphoethanolamine (NBD-PE) and octadecylrhodamine (R18) as fluorescent energy transfer donor and acceptor lipid probes, respectively, are mixed with non fluorescent liposomes and incubated in the absence or in the presence of oligomeric conjugates at various pH. Membrane fusion is evidenced by a decrease of the rhodamine fluorescence emission at 585 nm upon excitation at 470 nm, as a consequence of a decrease of the resonance energy transfer between NBD and rhodamine when the average spatial separation induced by membrane fusion increases.

The residues accounting for the destabilization of cellular membranes act through their property of being protonable in a weak acid medium.

The expression "weak acid medium" designates a medium the pH of which is lower than that of plasma or serum, i.e. a pH lower than 7.4.

Said medium can be either the extracellular medium or the lumen of intracellular compartments such as endosomes or lysosomes.

Said medium can be naturally acid or be acidified.

By way of example, the pH of said medium can be in the range of about 5 to about 7, in particular 5.5 to 6.5.

Transfer of the above-mentioned biomolecules into the cytosol and/or the cell nucleus, requires that both the oligomeric conjugate leading to the membrane destabilization and the above-mentioned biomolecules are present in the said medium.

In the present invention, the oligomeric conjugates and the above-mentioned biomolecules can be free or complexed.

Said positively charged oligomeric conjugate of the invention is liable to form a complex with at least one negatively charged oligoanion, the association between the oligoanion and the oligomeric conjugate being electrostatic in nature.

The expression according to which the protonable residues do not correspond to a recognition signal recognized by a cellular membrane receptor means that these residues are not used as ligands.

A molecule or a molecular complex is active as a recognition signal when it can be selectively recognized by a receptor, that is to say plays the role of a ligand, of an agonist, or of an antagonist. By recognition signal recognized by a cellular membrane receptor, one designates a ligand (molecule or molecular complexes) liable to be selectively recognized by said receptor.

According to an advantageous embodiment, the invention relates to a oligomeric conjugate, wherein the protonable residues leading to a destabilization of cellular membranes, present the additional properties :
- they are weak bases, the pK of which in aqueous medium is lower than 8, so that a proportion higher than 50 % of these bases linked to a cationic oligomer is not protonated at pH 7.4.

According to another advantageous embodiment, in the oligomeric conjugate of the invention, the protonable residues leading to a destabilization of cellular membranes, present the additional properties :
- they belong to the group of compounds comprising an imidazole ring,
- they belong to the group of quinolins,
- they belong to the group of pterins,
- they belong to the group of pyridins.

An example of quinolin is represented by the following formulae : 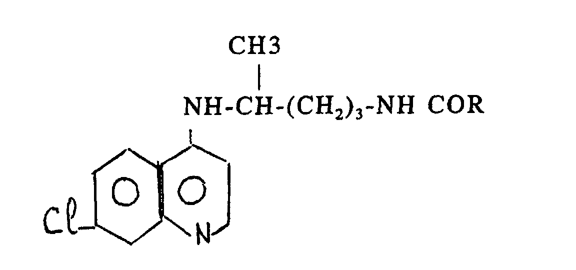 wherein R = (CH₂)ₙCO₂H, in which n varies from 1 to 10, preferably from 1 to 3.

Another example of quinolin is :

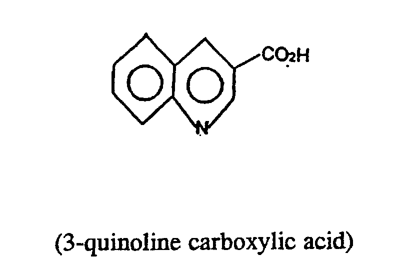

An example of pterin is represented by the following formula :

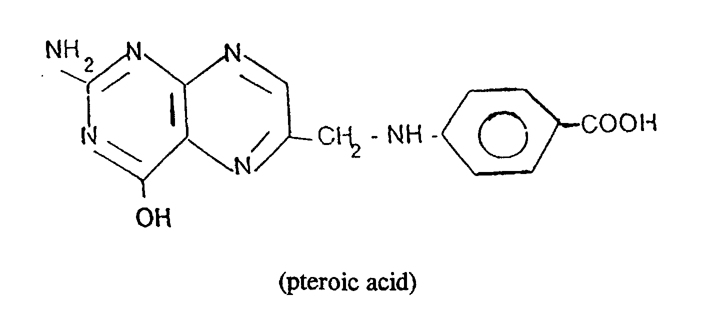

Examples of pyridins are represented by the following formulae :

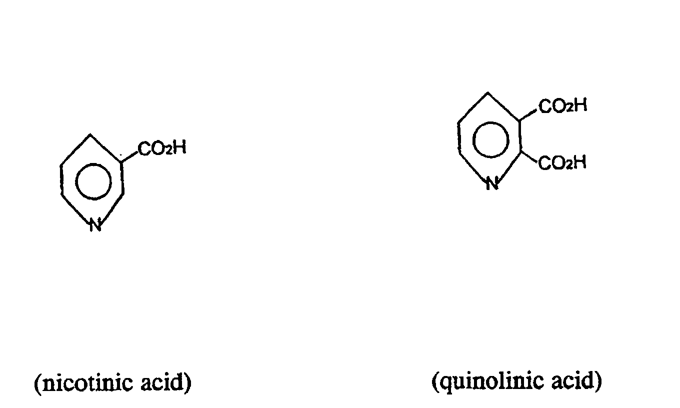

According to an advantageous embodiment, in the oligomeric conjugate of the invention, the protonable residues leading to a destabilization of the cellular membranes are :
- alkylimidazoles in which the alkyl radical comprises from 1 to 10, particularly from 2 to 6 carbon atoms, and in which only one of the nitrogen atoms of the imidazole ring is substituted.

According to an advantageous embodiment, in the oligomeric conjugate of the invention, the protonable residues leading to a destabilization of cellular membranes are chosen from :
histidine, 4-carboxymethyl-imidazole,
3-(1-methyl-imidazol-4yl)-alanine, 3-(3-methyl-imidazol-4yl)-alanine,
2-carboxy-imidazole, histamine, 3-imidazol-4yl)-L-lactic acid,
2-(1-methyl-imidazol-4yl)ethylamine, 2-(3-metyl-limidazol-4yl)ethylamine,
β-alanyl-histidine-(carnosine), 7-chloro-4(amino-1-methylbutylamino)-quinoline,
N⁴-(7-chloro-4-quinolinyl)-1,4-pentanediamine,
8-(4-amino-1-methylbutylamino)-6-methoxy-quinoline (primaquine),
N⁴-(6-methoxy-8-quinolinyl)1,4-pentanediamine, quininic acid,
quinoline carboxylic acid, pteroïc acid, nicotinic acid, quinolinic acid.

According to another advantageous embodiment, the oligomeric conjugate of the invention contains an oligomer of the following formula : 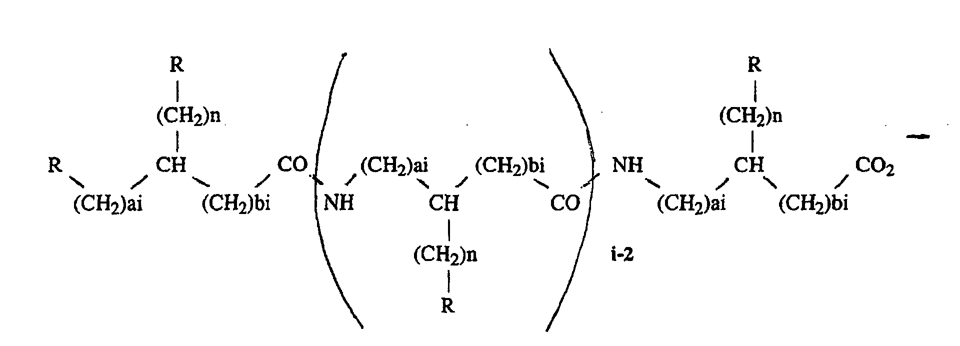 wherein
* ai is an integer varying from 0 to 10,
* bi is an integer varying from 0 to 10,
* i = degree of polymerization from 5 to 50, and particularly 10 to 40, and preferably 20,
* n = is an integer varying from 1 to 6, and preferably 4,
* R represents in a ratio of 50 % to 100 % (corresponding to a number u) 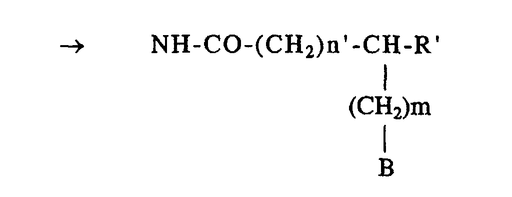 or 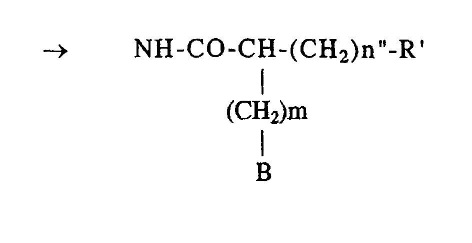 or 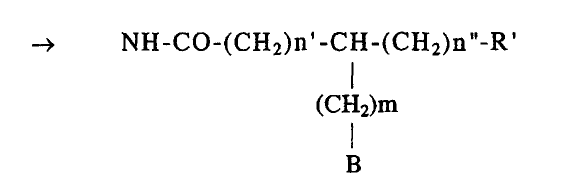
   - m: is an integer varying from 1 to 6,
   - n': is an integer varying from 0 to 6,
   - n": is an integer varying from 0 to 6,
   - B: is a weak base as defined above,
   - R': represents NH₃⁺ (corresponding to a number p),
   or NH (corresponding to a number q) substituted by
   -CO-CH₃
   -CO-(CHOH)rH r being from 1 to 15 preferably 2 to 7
   -CO-(CH₂)s-(CHOH)rH r being from 1 to 15 preferably 1 to 7 and s being from 1 to 6 preferably 4 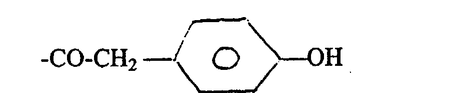 -SO₂-Flu
   - CO-Flu
   - CS-NH-Flu
   Flu being a fluorescent molecule
* R represents in a ratio of 0 % to 50 % (corresponding to f : 0 < f ≤ u)
   - NH₃⁺ (corresponding to a number j),
   - NH (corresponding to a number k), substituted by
      - CO-CH₃
      - CO-(CHOH)rH r being from 1 to 15 preferably 1 to 7
      -CO-(CH₂)s-(CHOH)rH r being from 1 to 15 preferably 1 to 7 and s being from 1 to 6 preferably 4 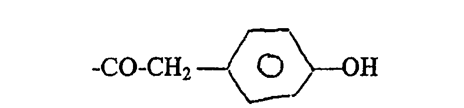 -SO₂-Flu
      - CO-Flu
      -CS-NH-Flu
      Flu being a fluorescent molecule
   - H (corresponding to a number h)
   - (CH₂) nH, n being an integer from 1 to 6 (corresponding to a number h)
   - (CH₂) n-OH n being an integer from 1 to 6 (corresponding to a number h)
   - (CH₂) n-SA' A' = H , CH₃ or 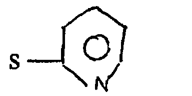
   n being integer from 1 to 6 (corresponding to a number h)
   with
   . i=u+j+k+h
   . total number of α NH₃⁺ = p = u-q
   . total number of ω NH₃⁺ = j = f-(k + h)
   . total number of NH₃⁺ = m = p + j + 1
   with the proviso that:
   1) u ≥ i/2
   2) m ≥ i/2

According to another advantageous embodiment, the oligomeric conjugate of the invention contains an oligomer of the following formula : 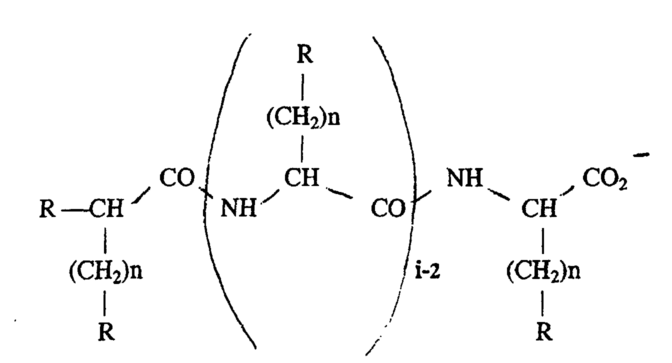 wherein
* i = degree of polymerization from 5 to 50, and particularly 10 to 40, and preferably 20,
* n = is an integer varying from 1 to 6, and preferably 4,
* R represents in a ratio of 50 % to 100 % (corresponding to u) 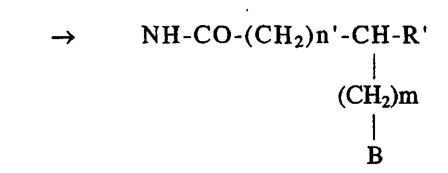 or 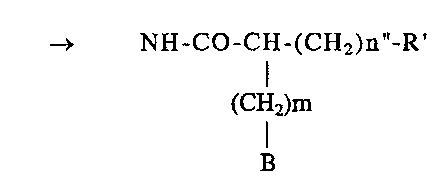 or 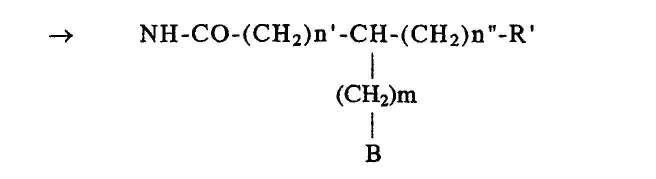
   - m: is an integer varying from 1 to 6,
   - n': is an integer varying from 0 to 6,
   - n": is an integer varying from 0 to 6,
   - B: is a weak base as defined above,
   - R': represents NH₃⁺ (corresponding to a number p),
   or NH (corresponding to a number q) substituted by
   -CO-CH₃
   - CO-(CHOH)rH r being from 1 to 15 preferably 1 to 7
   - CO-(CH₂)s-(CHOH)rH r being from 1 to 15 preferably 1 to 7 and s being from 1 to 6 and preferably 4 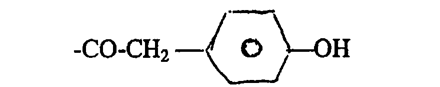 - SO₂-Flu
   - CO-Flu
   - CS-NH-Flu
   Flu being a fluorescent molecule
* R represents in a ratio of 0 % to 50 % (corresponding to f : 0 < f ≤ 1)
   - NH₃⁺ (corresponding to a number j),
   - NH (corresponding to a number k), substituted by
      - CO-CH₃
      - CO-(CHOH)rH r being from 1 to 15 preferably 1 to 7
      - CO-(CH₂)s-(CHOH)rH r and s being from 1 to 15 preferably 1 to 7 and s being from 1 to 6 and preferably 4 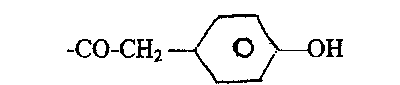 - SO₂-Flu
      - CO-Flu
      - CS-NH-Flu
      Flu being a fluorescent molecule
   - H (corresponding to a number h)
   - (CH₂)ₙH, n being an integer from 1 to 6 (corresponding to a number h)
   - (CH₂)ₙ-OH n being an integer from 1 to 6 (corresponding to a number h)
   - (CH₂)ₙ-SA' A' = H, CH₃ or 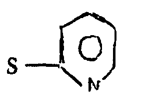
   n being integer from 1 to- 6 (corresponding to a number h)
   with
   . i = u + j + k + h
   . total number of α NH₃⁺ = p = u-q
   . total number of ω NH₃⁺ = j = f-(k + h)
   . total number of NH₃⁺ = m = p + j + 1
   with the proviso that :
   1) u ≥ i/2
   2) m ≥ i/2.

According to another advantageous embodiment, the oligomeric conjugate of the invention contains an oligomer of the formula above defined, wherein
i = 19 n = 4 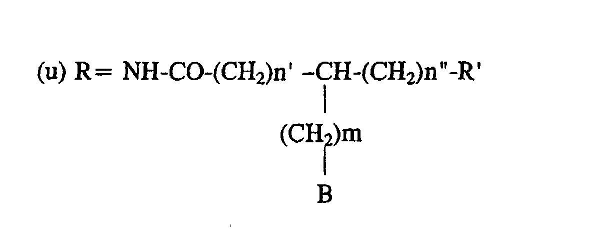 wherein
n' = n" = 0
R' = NH⁺₃
m = 1
B = imidazole
R =

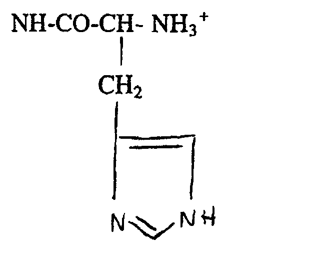

- (f) R: = NH⁺₃
- u: = 12
- j: = 7
or
i = 19 n = 4 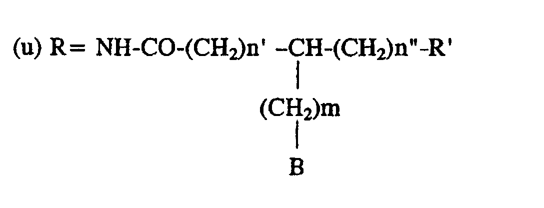 wherein
n' = n" = 0
R' = NH⁺₃
m = 1
B = imidazole
R =

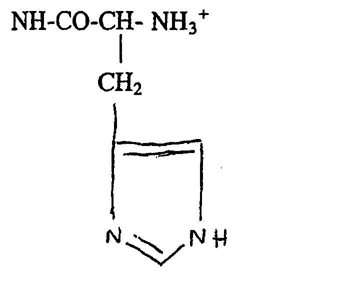

- (f) R: = NH⁺₃
- u: = 16
- j: = 3
or
i = 19 n = 4 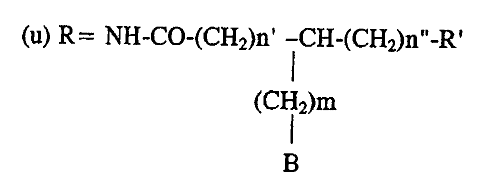 wherein
n' = n" = 0
R' = NH⁺₃
m = 1
B = imidazole
R =

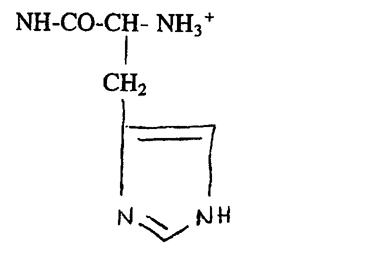

- (f) R: = NH⁺₃
- u: = 19
- j: = 0
or
i = 19 n = 4 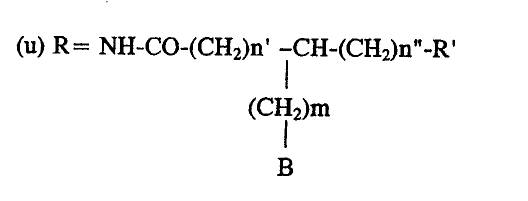 wherein
n' = n"=0
R' = NH⁺₃
m = 1
B = imidazole
R =

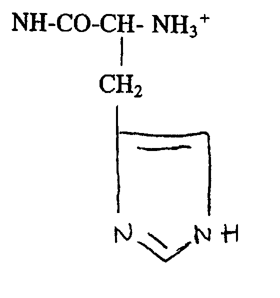

- (f) R: = CO-CH₃
- u: = 11
- k: = 8
or
i = 19 n = 4 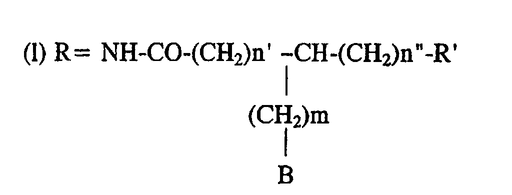 wherein
n' = n" = 0
R' = NH⁺₃
m = 1
B = imidazole
R =

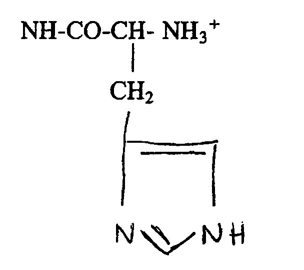

- (f) R: = CO-CH₃
- u: = 15
- k: = 4
or
i = 19 n = 4 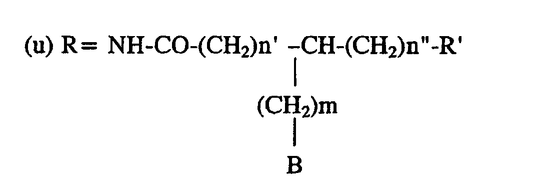 wherein
n' = n" = 0
R' = NH⁺₃
m = 1
B = imidazole
R =

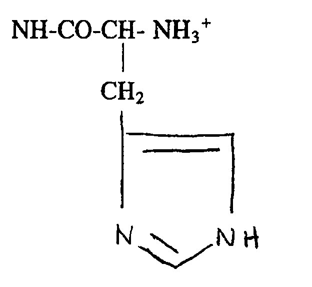

- (f) R: = CO-(CHOH)rH
- r: = 5
- u: = 12
- k: = 3
or
i = 19 n = 4 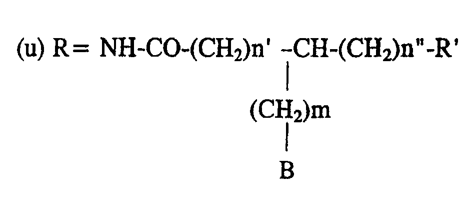 wherein
n' = n" = 0
(q) R' = NH-CO-CH₃
m = 1
B = imidazole
R =

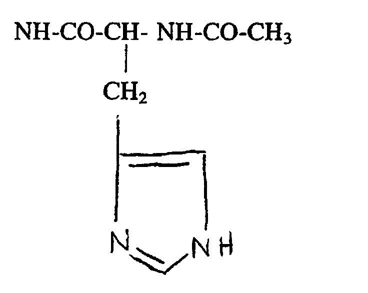

- (f) R: = NH⁺₃
- u: = 16
- f: = 4
- k: = 3

An example of mixed oligomer is the following : 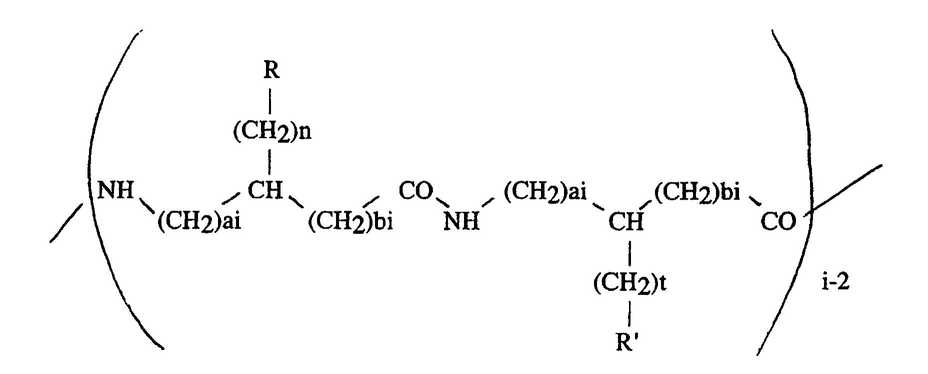 wherein
n=4 R' = H, CH₃, CH(CH₃)₂, CH₂,-CH(CH₃)₂ CH(CH₃)-CH₂-CH₃, CH₂OH, CHOH- CH₃, CH₂-CH₂-S- CH₃ (CH₂)n H, (CH₂)n-OH, (CH₂)n-SA'
A' = H, CH₃ or 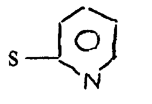
R = 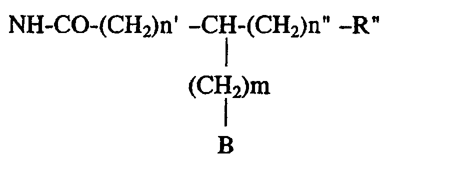
t varies from 1 to 6

As an example, when, in the above formula n = 4, ai = bi = 0, t = 1, R' = H, the monomers are lysine and valine.

The invention also relates to a composition containing a mixture of at least one oligomeric conjugate as defined above, with at least a peptide, an oligoside or an oligonucleotide derivative, or a mixture thereof.

In the composition of the invention, the oligomeric conjugates can be associated with a biological molecule, in particular an oligoanion, such as an oligonucleotide, an anionic peptide or an anionic oligoside, via electrostatic interactions.

An anionic oligoside can be a sulfated oligoside, succinylated oligoside, phosphorylated oligoside, sialylated oligoside or an oligoside containing pyruvilidenyl groups.

The invention also relates to a combined preparation containing as active substance the following individual components, in the form of a kit-of-parts :
- at least an oligomeric conjugate as defined above,
- at least as a peptide, an oligoside or an oligonucleotide, or a mixture thereof,
for the simultaneous, separate or sequential use, for the *in vitro,* or the *ex vivo* transfer of said biological molecules into the cytosol and/or the cell nucleus.

The invention also relates to a complex between at least one oligoanion which can be an anionic peptide, an anionic oligoside or an oligonucleotide or a mixture thereof, a mixture of at least one non negatively charged biological molecule and of at least one oligoanion, and at least one positively charged oligomeric conjugate as defined above, the association between the oligoanion and the oligomeric conjugate being electrostatic in nature.

According to another advantageous embodiment, the biological molecule is chosen among oligonucleotides, peptides, oligosaccharides or a mixture thereof.

An oligonucleotide used in the invention can be of the following formula : 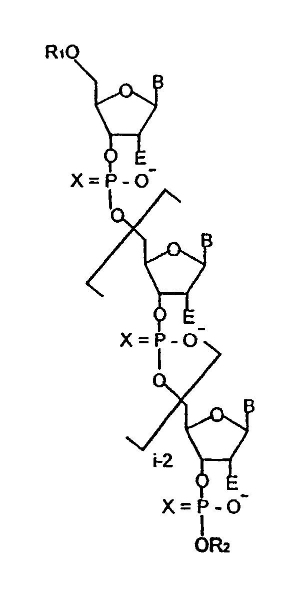 wherein *i* varies from 10 to 30, X represents O or S, B is a nucleic base U, A, T, G, C or a modified form such as a biotinyl or fluorescent labelled base which can be in α or β anomeric position, R1 and R2 represent independently from each other H, OH, (CH₂)n-A, [(CH₂) ₂-O] ₙ-CH₂-CH₂-A, A being H, OH, NH₂, COOH, 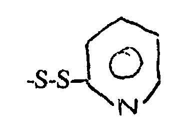 , n being an integer from 1 to 6,

E represents H, OH, OCH₃, OCH₂CH₃, O(CH₂)₂CH₃, O(CH₂)₃CH₃, O(CH₂)₄CH₃, O-CH₂-CH₂-O-CH₃

As example of oligonuclotides, one may cite the following :
GEM 91
   phosphorothioate (X = S) oligonucleotide i = 25 CTC TCG CAC CCA TCT CTC TCC TTC T complementary to the AUG initation site of gag HIV-1 gene
ISIS 1939
   phosphorothioate (X = S) oligonucleotide i = 19 CCC CCA CCA CTT CCC CTC T complementary to the 3' non coding region of ICAM-1 mRNA.

A mixture of an oligonucleotide and a peptide is defined as an oligonucleotide linked to a peptide, and a mixture of an oligonucleotide and an oligoside is defined as an oligonucleotide linked to an oligoside.

A mixture of an oligoside and a peptide is defined as an oligoside linked to a peptide.

A mixture of an oligonucleotide and a peptide or a mixture of an oligonucleotide and an oligoside used in the invention can have the following formulae : 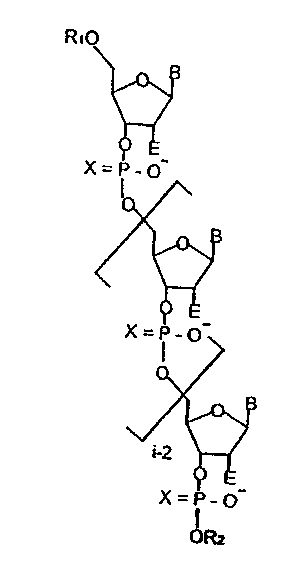 wherein *i* varies from 10 to 30, X represents O or S, B is a nucleic base U, A, T, G, C or a modified form such as a biotinyl or fluorescent labelled base which can be in α or β anomeric position, R1 and R2 represent independently from each other H, OH, (CH₂)n-A, [(CH₂) ₂-O] ₙ-CH₂-CH₂-A, A being H, OH, NH₂, COOH, 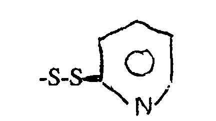 , n being an integer from 1 to 6, or a peptide or an oligoside,

E represents H, OH, OCH₃, OCH₂CH₃, O(CH₂)₂CH₃, O(CH₂)₃CH₃, O(CH₂)₄CH₃, O-CH₂-CH₂-O-CH₃

When R1 and/or R2 represent a peptide, the mixed oligoanion is a peptido-oligonucleotide, and when R1 and/or R2 represent an oligoside, the mixed oligoanion represents a glyco-oligonucleotide.

An example of oligonucleotide is a peptide nucleic acid (PNA) represented by the following formula : 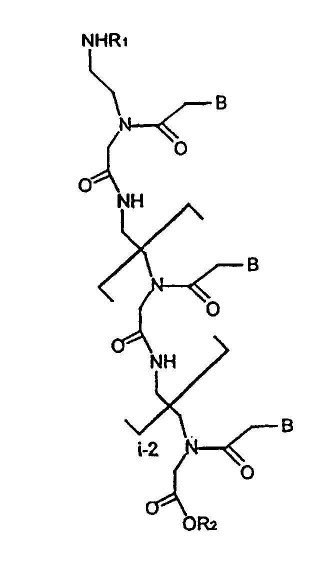 wherein - R1 and R2 represent independently from each other H, OH, (CH₂)n-A, [(CH₂) ₂-O] ₙ-CH₂-CH₂-A, A being H, OH, NH₂, COOH, 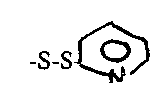 , n being an integer from 1 to 6,
- i is an integer varying from 10 to 30.

In the composition of the invention, the oligonucleotide can be single or double stranded, or can form a triplex (three strands) or a quadruplex (4 strands).

The invention also relates to the use of an oligomer conjugate as defined above, for the intracellular transfer of biological molecules into the cytosol or/and into the cell nucleus *in vitro* or *ex vivo.*

The invention also relates to the use of an oligomeric conjugate as defined above or of a composition as defined above, or of a combined preparation as defined above, for the preparation of a drug, for the intracellular *in vitro, ex vivo* or *in vivo* transfer of a peptide, an oligoside or an oligonucleotide, or a mixture thereof, into the cytosol or/and in to the cell nucleus of cells.

The invention also relates to the use of an oligomeric conjugate as defined above or of a composition as defined above, or of a combined preparation as defined above, wherein the cells are chosen among muscular, epithelial, endothelial, or myeloid cells such as monocytes, macrophages, fibroblasts, leukocytes and granulocytes, osteoblasts, as well as dendritic, stem, neuronal, or dermal cells, wherein the stem cells are not of human embryonic origin.

The invention also relates to a method for the *in vitro* or the *ex vivo* transfer of an oligonucleotide, wherein an oligonucleotide and an oligomeric conjugate as defined above, or of a composition as defined above, or of a combined preparation as defined above, are(is) contacted with a medium containing cells to be transferred, under conditions such that there is:
- transfer of an antisense oligonucleotide in the cytosol and/or the cell nucleus where it binds and blocks the complementary mRNA sequence,
- transfer of an oligonucleotide as activator into the cytosol where it depresses or activates a second messenger in the cytosol, or the corresponding gene in the nucleus,
- transfer into the cytosol and/or the cell nucleus of oligonucleotides corresponding to a repetitive bacterial type DNA sequence with stimulating or immunodepressive activity.

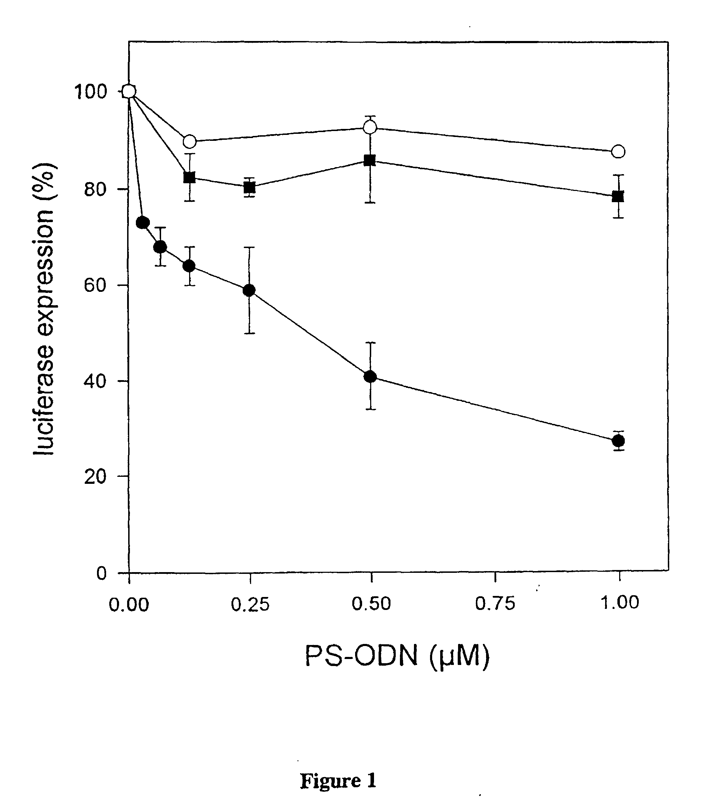

The transfer of an antisense oligonucleotide in the cytosol where it binds to the complementary mRNA sequence and blocks its traduction leading to inhibition of the synthesis of the gene product, can be carried out as described hereafter in the legends of Figures 1, 2 and 3.

The invention also relates to a method for the *in vivo,* the *in vitro* or the *ex vivo* transfer of an oligonucleotide, wherein an oligonucleotide and an oligomeric conjugate as defined above, or of a composition as defined above, or of a combined preparation as defined above, are(is) contacted with a medium containing cells to be transferred, under conditions such that there is :
- transfer into the cytosol and/or the cell nucleus of RNA or DNA oligonucleotide acting as decoys which inhibit gene expression by blocking the binding of regulatory factors to the authentic DNA region such as short RNA oligonucleotides corresponding to the HIV-TAR sequence inhibiting HIV expression and replication by blocking the binding of the HIV regulatory protein at Tat to the TAR region,
- transfer into the cytosol and/or the cell nucleus of ribozymes (RNA oligonucleotides) which inhibit gene expression by cleaving the mRNA.

The transfer into the nucleus of an oligonucleotide (triplex forming ODN, TFO) where it binds to target DNA at oligopurine sites where they form a triple-helical structure, leading to the inhibition of the gene expression, can be processed as hereafter described.

As an example of the specific TFO is an oligonucleotide 5'-A₄GA₄G₆A-3' directed against the polypurine track (PPT) in the NEF-HIV-1 gene.

The transfer of the oligonucleotide as activator of the immune response can be carried out as follows :

As an example the double strand RNA polyinosinic-polycytidylic acid (poly I:C) for the increase of the tumoricidal activity of macrophages or for the stimulation of natural killer lymphocyte cytotoxicity.

The transfer of double stranded polynucleotide as activator of the immune response can be illustrated by the following :

Polyinosinic-polycytidylic acid (poly I:C) is known to increase the tumoricidal activity of macrophages *via* a TNF-α mediated cytolysis.

Thioglycolate-elicited peritoneal macrophages (2.5 x 10⁵) are plated in 16 mm diameter well multiwell plates in serum free RPMI medium. Upon 2 h incubation at 37°C, non-adherent cells are discarded. Adherent cells are cultured in 0.5 ml of serum free RPMI medium in the absence or the presence of polyI:C and in the absence or the presence of histidylated oligolysine. Culture supernatants are collected after 24 h incubation at 37°C. Before testing, supernatants are rendered cell-free by centrifugation at 2000 g for 10 minutes. The cytotoxic activity of macrophage culture supernatants is determined by using L929 cells pretreated for 2 h at 37°C with 2 µg/ml actinomycin D. Actinomycin D pretreated L929 cells are seeded in 96-wells microplates (4 x 10⁴ cells in 0.05 ml serum free RPMI medium per well). After 3-4 h at 37°C, diluted supernatants from stimulated macrophages are added (0.05 ml) and the cells are incubated at 37°C for 18-20 h. Microplates are washed with PBS and the percentage of cell lysis is determined after staining the cells with 0.05 ml of crystal violet (0.2% in 2 % ethanol). The plates are washed with tap water and the dye is solubilized by adding 0.06 ml per well of 0.3 % sodium dodecyl sulfate. Absorbance of each well is read at 570 nm. The % cytotoxicity is calculated according to (A_{NS} - A_{S})/A_{NS} x 100 where A_{NS} and A_{S} are the absorbances of wells containing target cells incubated with supernatant dilutions of non stimulated and stimulated macrophages, respectively.

The invention also relates to a method for the *in vivo, the in vitro* or the *ex vivo* transfer of peptide, wherein a peptide and an oligomeric conjugate as defined above, or of a composition as defined above, or of a combined preparation as defined above, are(is) contacted with a medium containing cells to be transferred, under conditions such that there is a transfer of said peptide into the cytosol.

The transfer of a peptide can be illustrated by the following :
A) Cells are incubated for 4 h at 37°C with 1 µM fluorescein-labelled peptide (*F-S-*CGEEDTSEKDEL) in the absence or in the presence of histidylated oligolysine. Cells are fixed with 2 % of p-formaldehyde, washed and mounted on slides in a PBS/glycerol mixture (1:1 v/v) containing 10 mg/ml DABCO (1,4 diazobicyclo-(2,2,2)-octane) as antifading agent. Cells are analyzed with a confocal microscope imaging system (MRC-1024, Bio-Rad) equipped with a Nikon Optiphot epifluorescence microscope.
B) Dendritic cells are incubated for 4 h at 37°C with 1 µM c-myc epitope peptide (SMEQKLISEEDLNFELDEA) in the absence or in the presence of histidylated oligolysine. Cells are fixed with 2 % of p-formaldehyde in the presence of 0.5 % saponine, washed and then incubated for 1 h with anti c-myc epitope monoclonal antibody (9E10) in PBS containing containing 10 mg/ml BSA and 0.1 % saponin. Cells are washed and further incubated for 1 h in the presence of fluorescein-labelled anti-mouse IgG F(ab)' fragments in PBS containing containing 10 mg/ml BSA and 0.1% saponin. Cells are washed and mounted on slides in a PBS/glycerol mixture (1:1 v/v) containing 10 mg/ml DABCO (1,4 diazobicyclo-(2,2,2)-octane) as antifading agent. Cells are analyzed with a confocal microscope imaging system (MRC-1024, Bio-Rad) equipped with a Nikon Optiphot epifluorescence microscope.

The fixation of a peptide to intracellular cofactor can be carried as described in the following example:

An oligopeptide corresponding to a prostate specific antigen (PSA) epitope mixed to the oligomeric conjugate is transferred into the cytoplasm of macrophages.

The oligopeptide is fixed there to heat shock proteins (HSP90, HSP70) to form HSP-peptide complexes which are then re-expressed at the surface of macrophages. This complex formed with the HSP cofactor stimulate macrophages and enhance the immune response to the PSA antigen.

The invention also relates to a method for the *in vivo,* the *in vitro* or the *ex vivo* transfer of peptide, wherein a peptide and an oligomeric conjugate as defined above, or a composition as defined above, or a combined preparation as defined above, in particular wherein the peptide is an antigenic peptide, are(is) contacted with a medium containing cells to be transferred, under conditions such that there is a transfer of said antigenic peptide in the cytosol of antigen presenting cells (macrophages, dendritic cells and B cells) where they are processed in proteosomes in order to bind to MHCI molecules, allowing the presentation of the antigenic epitope fixed on MHCI.

*In vitro* evaluation of antigen presentation can be illustrated by the following:

Dentritic cells were incubated for 4 h at 37°C with the nonadecapeptide (185-203) from the C-terminal part of the HIV-1 Nef protein containing the nonapeptide (190-198) (AFHHVAREL) in the absence or in the presence of an histidylated oligolysine. Cells are washed and further incubated for 24 h at 37°C in the absence of peptide and histidylated oligolysine. MHC class I presentation of peptide antigen was evaluated by Cr⁵¹ cytotoxic assay by using a CTL clone sensible to the peptide. DCs were labelled with Cr⁵¹ (target cells : T) and then incubated at 37°C for 4 h in the presence of the CTL clone (effector cells : E) at E/T ratios ranged from 1 to 100. The supernatants are collected and the radioactivity in the supernatant was recorded. The % of specific Cr⁵¹ release is calculated according to (A_{NS} - A_{S})/A_{NS} x 100 where A_{NS} and A_{S} are the radioactivity in supernatant dilutions of dentritic cells incubated in the absence and the presence of CTL cells, respectively.

The invention also relates to a method for the *in vitro* or the *ex vivo* transfer of a oligoside, wherein an oligoside and an oligomeric conjugate as defined above, or a composition as defined above, or a combined preparation as defined above, are(is) contacted with a medium containing cells to be transferred, under conditions such that there is a transfer of said oligoside into the cytosol and/or the cell nucleus.

An example of transfer of an oligoside can be illustrated by the following :

Cells are incubated for 4 h at 37°C with 0.5 mg/ml fluoresein-labelled dextrans (either Mw 4000 or Mw 70000) in the absence or in the presence of an oligomeric conjugate. Cells are washed with PBS, fixed with 2 % of p-formaldehyde, washed and mounted on slides in a PBS/glycerol mixture (1:1 v/v) containing 10 mg/ml DABCO (1,4 diazobicyclo-(2,2,2)-octane) as antifading agent. Cells are analyzed with a confocal microscope imaging system (MRC-1024, Bio-Rad) equipped with a Nikon Optiphot epifluorescence microscope (Nikon, Tokyo, Japan) and a planapo objective (numerical aperture 1.4).

An example of transfer of a negatively charged oligoside can be illustrated by the following :

Cells are incubated for 4 h at 37°C with 0.5 mg/ml fluoresein-labelled polyanionic dextrans (either Mw 3000 or Mw 70000) in the absence or in the presence of an oligomeric conjugate. Cells are washed with PBS, fixed with 2 % of p-formaldehyde, washed and mounted on slides in a PBS/glycerol mixture (1:1 v/v) containing 10 mg/ml DABCO (1,4 diazobicyclo-(2,2,2)-octane) as antifading agent. Cells are analyzed with a confocal microscope imaging system (MRC-1024, Bio-Rad) equipped with a Nikon Optiphot epifluorescence microscope (Nikon, Tokyo, Japan) and a planapo objective (numerical aperture 1.4).

The fixation of an oligoside to intracellular cofactor can be carried as described in the following example:

An oligoanion with silicated saccharidic components complexed according to the invention is transported into the cytoplasm of human cells where it binds to intracellular cofactors or second messengers such as NF kappa B. This binding causes nuclear transfer of the cofactor which derepress or stimulates genes coding for cytokines (such IL1, TNF-α, IL-12).

This results in a marked stimulation of the human cell cultured in the presence of the complex.

The invention also relates to a pharmaceutical composition, comprising as active substance at least an oligomeric conjugate as defined above, or a composition as defined above, or a combined preparation as defined above, or in association with a pharmaceutically acceptable vehicle.

The invention also relates to the use of an oligomeric conjugate as defined above, or of a composition as defined above, or of a combined preparation as defined above, or for the preparation of a drug for use in the treatment of cancer, inflammatory or immunology diseases (such as graft rejection, allergy, auto-immunity) or infectious diseases.

The invention also relates to a kit or case containing :
- an oligomeric conjugate as defined above, substituted by a protonable residue leading in a weak acid medium to a destabilization of cellular membranes, this oligomeric conjugate being able to comprise a recognition signal, which is previously fixed or not on the above-said conjugate, said recognition signal being dependent upon the cell to target,
- at least one biological molecule to transfer,
- optionally reagents enabling the possible binding of the recognition signal on the above-said oligomeric conjugate,
- optionally reagents enabling the formation of a composition as defined above, or of a combined preparation as defined above,
- reagents enabling the transfer of the biological molecule in the cytosol and/or the cell nucleus.

### LEGEND OF THE FIGURES

### Inhibition of luciferase gene expression by GEM-91.

Figure 1 shows the activity of GEM-91, an antisense phosphorothioate oligonucleotide (PS-ODN) (CTC TCG CAC CCA TCT CTC TCC TTC T) complementary to the AUG initiation site of gag HIV-1 gene. The effect of histidylated oligolysines was evaluated by using pRET-Luc cells (a rabbit smooth muscle cell line). These cells produce endogenous luciferase under the control of the human phosphoglycerate kinase promoter and the luciferase gene sequence around the AUG codon was replaced by the initiator AUG codon and several downstream codons of gagHIV-1 gene. The results showed that the activity of GEM-91 (IC₅₀ > 5 µM) was increased more than 10 times in the presence of 20 µM HoK2 (IC₅₀ 0.25 µM). Whilst, no significant inhibition was obtained in the presence of HoK3 in which the α-NH₂ histidyl residues were acetylated, suggesting that interactions between ODN and histidylated oligolysines were involved. pRET-Luc cells, seeded onto 24-well plates (2 x10⁵ cells/well), were treated for 4 h at 37°C in DMEM supplemented with 2 % FBS containing various concentrations of GEM-91, (■) in the absence of histidylated oligolysine, (●) in the presence of 20 µM HoK2 or (○) in the presence of 20 µM HoK3. HoK2 and HoK3 are histidylated oligolysines prepared as described in the following text. Then, FBS was raised to 6 % and cells were further incubated for 18 h. Luciferase gene expression was measured by recording luminescence for 4 s. The percentage of luciferase inhibition was calculated by using , [(RLU^{ODN} -RLU)/RLU] x 100 where RLU^{ODN} and RLU were the luciferase activity into cell lysates of cells incubated in the absence and in the presence of ODN, respectively. Results shown typical of experiments carried out in triplicate and repeated at least twice. Data are means ± standard deviation.

### Inhibition of TNF-α induced ICAM-1 expression by ISI 1939.

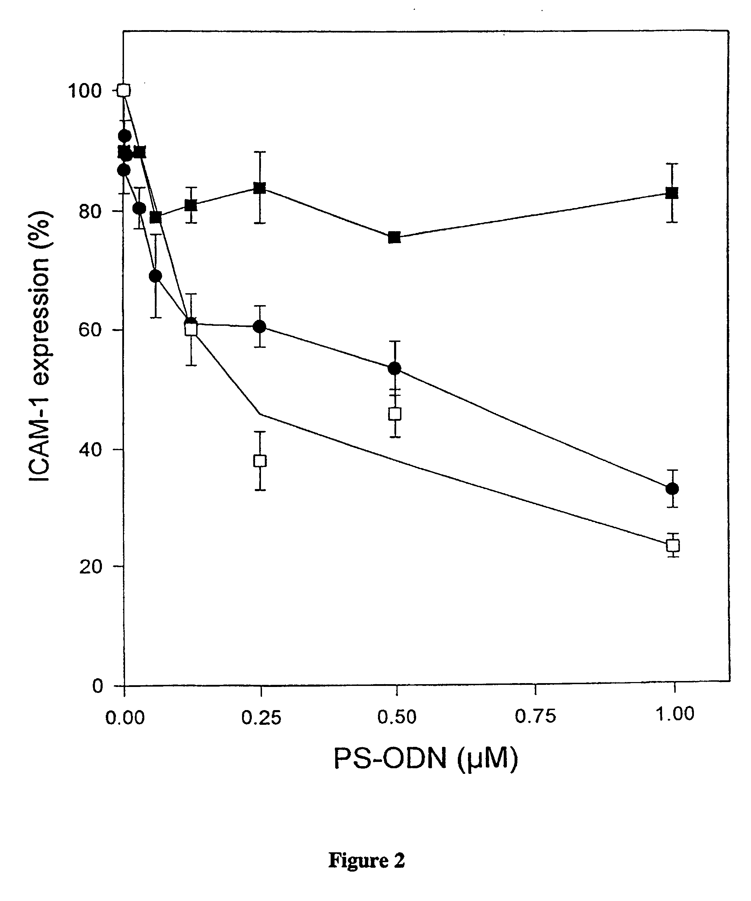

Figure 2 shows the inhibitory effect of TNF-α induced ICAM-1 expression by ISIS 1939 (CCCCCACCACTTCCCCTCT), an antisense phosphorothioate oligonucleotide (PS-ODN) targeted to the 3' non-coding region of ICAM-1 mRNA. The results showed that TNF-α induced ICAM-1 expression was inhibited by ISIS 1939 in the presence of 20 µM of histidylated oligolysines. HoK2 (IC₅₀ of 0.25 µM) appeared to be more efficient than HoK1 (IC₅₀ of 0.5 µM) probably because HoK2 bore less histidyl residues than HoK1 (15 *versus* 12). The inhibition was very low in the absence of histidylated oligolysines even up to 1 µM ODN (20 % inhibition). A549 cells (ATCC CCL 185, ATCC Rockville, MD) were plated onto 96-wells microtiter plates (10⁴ cells /well). The day after, culture medium was removed and cells were washed. Cells were incubated at 37°C for 4 h in 100 µl DMEM serum-free medium containing ISIS 1939 ODN either in the absence (■) or in the presence of 20 µM (●) HoK1 or (□) HoK2. HoK1 and HoK2 are histidylated oligolysines prepared as described in the following text. One volume of fresh medium containing 10 ng/ml TNF-α was added and cells were further incubated for 18 h. ICAM-1 expression was quantified by ELISA using anti-ICAM-1 antibodies. Cells were washed 3 times with 200 µl of PBS and fixed for 20 min at room temperature in PBS containing 20 mg/ml paraformaldehyde. Then, cells were incubated for 90 min at 37°C with anti-ICAM 1 mouse antibody (Becton Dickinson) diluted 20 times in PBS containing 20 mg/ml BSA. Cells were washed 3 times with PBS and then incubated for 1 h at 37°C with an anti-mouse horseradish peroxydase conjugate (Becton Dickinson) diluted 2000 times in PBS containing 20 mg/ml BSA. After 3 whashes, the peroxydase activity was assessed by using 100 µl of o-phenylenediamine dihydrochloride peroxydase substrate tablet set (Sigma). After X min incubation at 37°C, the reaction was stopped by adding 25 µl of 3 N H₂SO₄ and the absorbance read at 492 nm. All calculations were made relative to untreated controls in the absence or in the presence of TNF-α. The percentage of TNF-α-induced expression of ICAM-1 was calculated as follows : [(A_{TNF-α} ^{ODN} - A₀) / (A_{TNF-α}-A₀)] x 100 where A_{TNF-α} ^{ODN} was the absorbance of ODN treated and cytokine-induced cells, A₀ the absorbance of cells incubated without ODN and TNF-α, and A_{TNF-α} the absorbance of cytokine-induced cells incubated without ODN. Results shown are typical of experiments carried out in triplicate and repeated at least twice. Data are means ± standard deviation of the percentage of control ICAM-1 expression induced by TNF-α.

### Effect of histidylated polymers on the intracellular location of PS-ODN.

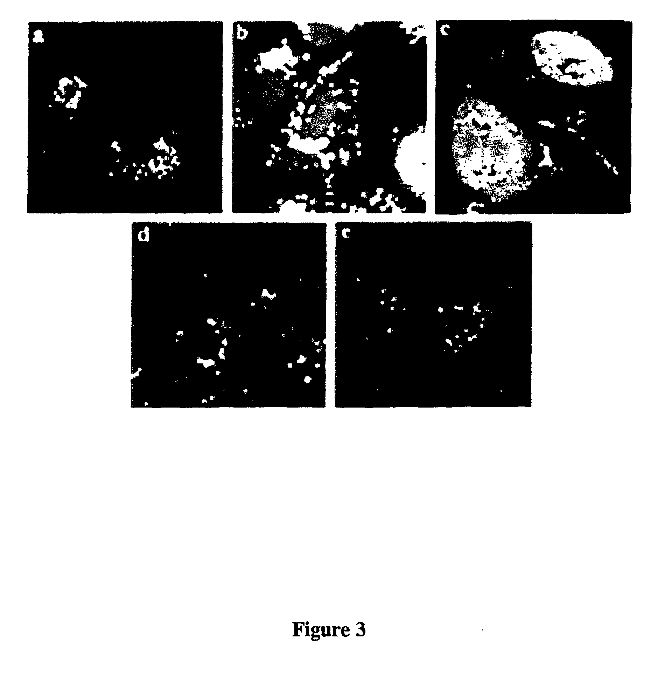

Figure 3 shows that histidylated oligolysines induced cytosolic and nuclear delivery of ODN. A549 cells incubated for 4 h at 37°C with 0.125 µM *F*-PS-ODN in the absence of histidylated oligolysine exhibited a faint vesicular staining (Fig 3-a). In the presence of either HoK1 (Fig 3-b) or HoK2 (Fig 3-c), the fluorescent staining was more intense in agreement with the flow cytometry analysis. HoK1, HoK2 and HoK3 are histidylated oligolysines prepared as described in the following text. In addition, the vesicles appeared bigger and the cytosol and the nucleus were also labelled while vesicles were smaller and neither the cytosol and the nuclear were staining in the absence of histidylated oligolysine (Fig 3-a). The cytosolic and nuclear staining was greater in the presence of HoK2 than in the presence of HoK1, probably because HoK2 contained more histidyl residues than HoK1 (15 *versus* 12). In contrast, the cell associated fluorescence was low in the presence of HoK3 suggesting that interactions between ODN and HoK1 and HoK2 might favor the ODN uptake (Fig 3-d). Unsubstituted oligolysine had no effect on the ODN uptake and ODN accumulation (Fig 3-e). A549 cells (ATCC CCL 185, ATCC Rockville, MD) were seeded onto sterile coverslips in 20-mm wells (2 x 10⁵ cells/ well) and allowed to adhere. Cells were incubated in the presence of 0.125 µM fluorescein-labelled PS-ODN for 4 h at 37°C (a) in the absence or in the presence of 20 µM (b) HoK1, (c) HoK2, (d) HoK3 or (e) Plk (Oligolysine containing 19 lysyl residues and non substituted by histidine used as control). Cells were fixed with 2 % of p-formaldehyde, washed and mounted on slides in a PBS/glycerol mixture (1:1 v/v) containing 10 mg/ml DABCO (1,4 diazobicyclo-(2,2,2)-octane) as antifading agent. Cells were analyzed with a confocal microscope imaging system (MRC-1024, Bio-Rad) equipped with a Nikon Optiphot epifluorescence microscope (Nikon, Tokyo, Japan) and a planapo objective (numerical apErture 1.4).

### EXAMPLES

### Preparation of histidylated oligolysines :

Oligolysine (Poly-L-lysine, HBr ; average molecular weight of 3950 ; average degree of polymerization of 19) (Bachem Feinchemikalien, Bubendorf, Switzerland) (1 g in 200 ml H₂O) was passed through an anion exchange column (Dowex 2 x 8, ⁻OH form, 20-50 mesh) in order to remove bromide ions. The eluate was neutralized with a 10 % p-toluene sulfonic acid solution in water and freezed-dried.

### Example 1 : Preparation of HoK1

Oligolysine *p*-toluene sulfonate salt (50 mg ; 8.6 µmol) in 2 ml dimethylsulfoxide (Aldrich, Strasbourg, France) in the presence of diisopropylethylamine (50 µl ; 344 µmol) (Aldrich) was reacted for 20 h at 20°C with (Boc)His(Boc)-OH (64 mg ; 146 µmol) (Novabiochem, Bad Soden, Germany) in the presence of benzotriazol-1-yl-oxy-tris-(dimethylamino) phosphonium hexafluorophosphate (BOP) (Richelieu Biotechnologies, Saint Hyacinthe, Canada) (159mg ; 358 µmol). The residual ε-amino groups of oligolysine was then substituted with gluconoyl residues (GlcA) : δ-gluconolactone (86mg ; 48 µmol) (Aldrich) and diisopropylethylamine (134 µl; 921 µmol) were added and the solution was stirred for 20h at 20°C. The *N*-protecting Boc groups were removed by acidic treatment by adding 10 volumes of a H₂O/trifluoroacetic acid mixture (1:1 ; v/v) for 24h at 20°C. Water and trifluoroacetic acid were removed under reduced pressure. HoK1 was precipitated by adding 10 volumes of isopropanol and spun down by centrifugation (1800g for 15 min). The pellet was washed with isopropanol, collected by centrifugation (1800g for 15 min), solubilized in distilled water and freezed-dried. The average number of histidyl residues bound per oligolysine molecule was determined by ¹H-NMR spectroscopy at 300 MHz in D₂O according to : x = 6. (h_{8.7} / h_{Lys}) . DP, where h_{8.7} was the value of the integration of the signal at 8.7 ppm corresponding to the proton (1H C₁₂) of histidyl residues, h_{Lys} that in the range from 1.3 to 1.9 ppm corresponding to the 6 methylene protons (C₃, C₄ and C₅) of lysyl residues and DP the degree of polymerization of oligolysine. The number of histidyl residues bound per oligolysine molecule was 12. The average number of gluconoyl residues bound per oligolysine molecule was determined by ¹H-NMR spectroscopy from : x = 3/2. (h_{G} / h_{Lys}) . DP, where h_{G} was the value of the integration in the range 3.6 to 3.9 ppm of the 4 protons (1H C₁₀, 1H C₁₁ and 2H C₁₂) of gluconoyl residues, h_{Lys} that in the range of 1.3 to 1.9 ppm of the 6 methylene protons (C₃, C₄ and C₅) of lysyl residues and DP the degree of polymerization of pLK. The number of gluconoyl residues bound per oligolysine molecule was 3. The number of free ε-amino groups per oligolysine molecule was 4.

### Example 2 : Preparation of HoK2

Oligolysine *p*-toluene sulfonate salt (80 mg ; 13.7 µmol) in 3 ml dimethylsulfoxide in the presence of diisopropylethylamine (90 µl; 620 µmol) was reacted for 6 h at 20°C with the N-hydroxysuccinimidyl derivative of (Boc)His(Boc)-OH (92 mg ; 204 µmol) (Bachem Feinchemikalien). The residual ε-amino groups of oligolysine was then acetylated (Ac) : acetic anhydride (13 µl ; 109 µmol) (Aldrich) and diisopropylethylamine (5 µl; 35 µmol) were added and the solution was stirred for 30 min at 20°C. The *N*-protecting Boc groups were removed by acidic treatment by adding 10 volumes of a H₂O/trifluoroacetic acid mixture (1 : 1 ; v/v) for 2 h at 20°C. Water and trifluoroacetic acid were removed under reduced pressure. HoK2 was precipitated by adding 10 volumes of isopropanol and spun down by centrifugation (1800 g for 15 min). The pellet was washed with isopropanol, collected by centrifugation (1800 g for 15 min), solubilized in distilled water and freezed-dried. The average number of histidyl residues bound per oligolysine molecule was determined by ¹H-NMR spectroscopy at 300 MHz in D₂O according to : x = 6 . (h_{8.7} / h_{Lys}). DP, where h_{8.7} was the value of the integration of the signal at 8.7 ppm corresponding to the proton (1H C₁₂) of histidyl residues, h_{Lys} that in the range from 1.3 to 1.9 ppm corresponding to the 6 methylene protons (C₃, C₄ and C₅) of lysyl residues and DP the degree of polymerization of oligolysine. The number of histidyl residues bound per oligolysine molecule was 15. The average number of acetyl residues bound per oligolysine molecule was determined by ¹H-NMR spectroscopy from : x = (h_{A} / h_{Lys}). DP, where h_{A} was the value of the integration at 2.04 ppm of the 3 protons of acetyl residues, h_{Lys} that in the range of 1.3 to 1.9 ppm of the 6 methylene protons (C₃, C₄ and C₅) of lysyl residues and DP the degree of polymerization of pLK. The number of acetyl residues bound per oligolysine molecule was 3. The number of free ε-amino groups per oligolysinemolecule was 1.

### Example 3 : Preparation of HoK3

Oligolysine *p*-toluene sulfonate salt (85 mg ; 14.6 *µ*mol) in 3 ml dimethylsulfoxide in the presence of diisopropylethylamine (80 *µ*l; 555 µmol) was reacted for 20 h at 20°C with N-acetyl-His-OH (288 mg ; 307 *µ*mol) (Sigma) in the presence of BOP (265 mg ; 597 *µ*mol). The residual ε-amino groups of oligolysine was then acetylated (Ac) with acetic anhydride for 30 min at 20°C. HoK3 was precipitated by adding 10 volumes of isopropanol and spun down by centrifugation (1800 g for 15 min). The pellet was washed with isopropanol, collected by centrifugation (1800 g for 15 min), solubilized in distilled water and freezed-dried. The average number of histidyl residues bound per oligolysine molecule was determined by ¹H-NMR spectroscopy as describe The number of histidyl residues bound per oligolysine molecule was 15. The number of free ε-amino groups per oligolysine molecule was 1.

### Example 4 : Preparation of synthons for synthesis of oligomeric conjugates

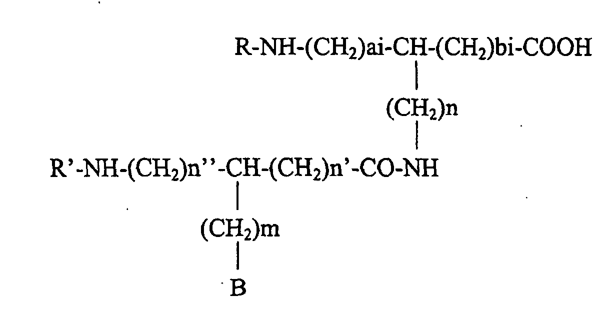

wherein
R and R' represent aminoprotecting groups, ai is an integer varying from 0 to 6, bi is an integer varying from 0 to 6, n is an integer varying from 1 to 6, n' is an integer varying from 0 to 6, n" is an integer varying from 0 to 6, m is an integer varying from 1 to 6.

An example of synthon : the Lys(His) synthon
wherein
ai = bi = 0
n = 4
R = Fmoc
n' = n"= 0
m = 1
R' = Boc
B = (NBoc)Imidazole

The N-hydroxysuccinimidyl derivative of (Boc)₂-His-OH [(Boc)₂-His-OSu] (1g ; 2.2 mmol) is coupled to Fmoc-Lys-OH (722 mg ; 2.2 mmol) for 24 h at 20°C in dimethylformamide (ml). The Lys(His) synthon is precipitated with isopropanol, collected by centrifugation, washed with ether and dried under vacuum. The Lys(His) synthon is purified by cristallization.

### Example 5 :

### Preparation of an histidylated oligolysine (HoK) containing 20 lysine residues and 20 histidyl residues.

A HoK containing exactly 20 lysyl residues and 20 histidyl residues can be entirely synthetised by using the above Lys (His) synthon. Briefly, 20 Lys(His) synthon are successively assembled on a Applied Biosystems 433A synthesizer with conductimetric monitoring by using Fmoc-protected amino acids. Lys(His) synthons are coupled by the HBTU activation method. HoK are cleaved from the resin and side chain protecting Boc groups are removed with a trifluoroacetic acid/water mixture (50% : 50% ; v/v) for 3 h at room temperature. Crude HoK is precipitated with isopropanol and collected by centrifugation. HoK is washed three times with isopropanol, resuspended in distilled water and freezed-dried.

### Example 6

### Preparation of an oligomeric conjugate containing 17 lysyl residues substituted with 17 histidyl residues and 3 leucyl residues inserted any where in the lys (His) sequence

Oligomers made of exactly 17 lysyl residues substituted with 17 histidyl residues and 3 leucyl residues insert anywhere in the Lys(His) sequence can be entirely synthetised by using the above Lys (His) synthon and Fmoc-Leu on a Applied Biosystems 433A synthesizer with conductimetric monitoring by using Fmoc-protected amino acids. Lys(His) synthons and Leu were coupled by the HBTU activation method. Oligomers are cleaved from the resin and side chain protecting Boc groups are removed with a trifluoroacetic acid/water mixture (50% : 50% ; v/v) for 3 h at room temperature. Oligomers are precipitated with isopropanol and collected by centrifugation. Oligomers are washed three times with isopropanol, resuspended in distilled water and freezed-dried.

### Example 7 : Preparation of (K(His)-KL(His)-L)₇

An oligomer (K(His)-K(His)-L)₇ can be entirely synthetised by using the above Lys (His) synthon and Fmoc Leu on a Applied Biosystems 433A synthesizer with conductimetric monitoring by using Fmoc-protected amino acids. Lys(His) synthons and Leu are coupled by the HBTU activation method. The oligomer (K(His)-K(His)-L)₇ is cleaved from the resin and side chain protecting Boc groups are removed with a trifluoroacetic acid/water mixture (50% : 50% ; v/v) for 3 h at room temperature. The polymer is precipitated with isopropanol and collected by centrifugation. The oligomer is washed three times with isopropanol, resuspended in distilled water and freezed-dried.

### Example 8 : Preparation of(K(His)-L-K(His))₇

A oligomer (K(His)-L-K(His))₇ can be entirely synthetised by using the above Lys (His) synthon and Fmoc Leu on a Applied Biosystems 433A synthesizer with conductimetric monitoring by using Fmoc-protected amino acids. Lys(His) synthons and Leu are coupled by the HBTU activation method. The oligomer is cleaved from the resin and side chain protecting Boc groups are removed with a trifluoroacetic acid/water mixture (50% : 50% ; v/v) for 3 h at room temperature. The oligomer is precipitated with isopropanol and collected by centrifugation. The oligomer is washed three times with isopropanol, resuspended in distilled water and freezed-dried.

Table I shows that oligolysine having a DP of either 190, 72 or 36 substituted with less that 45% of histidyl residues, do not allow the transfer of small nucleic acid, particularly oligonucleotides. Indeed, it is necessary to adapt the size of histidylated oligolysines. Conversely, small histidylated oligolysines (DP of 36 or 19) substituted by more than 50% of histidyl residues do not allow efficient gene transfer by histidylated oligolysine/plasmid complexes (Table I). In addition oligolysines substituted with less than 50% histidyl residues are cytotoxic.

**Table I :**

| comparative evaluation of gene transfer and oligonucleotide transfer by using histidylated polylysine. | | | | |
|---|---|---|---|---|
| DP | His (%) | DNA | ODN | Cytotoxicity (%) |
| 190 | 35 | 100 | 0 | 24 |
| 190 | 45 | 110 | 0 | 21 |
| 72 | 23 | 87-96 | 0 | 24 |
| | | | | |
| 36 | 22 | 61-100 | 0 | 25 |
| 36 | 53 | 0-10 | 20 | 4 |
| 19 | 25 | 15-26 | 0 | 49 |
| 19 | 45 | 9 | 0 | 40 |
| 19 | 60 | nd | 100 | 26 |
| 19 | 80 | nd | 100 | 0 |
| 19 | 100 | nd | 100 | 0 |

DP is the oligolysine degree of polymerization. DNA corresponds to transfection by using histidylated oligolysine/pCMVLUC. The transfection efficiency is scored on a 0 to 100 scale. The transfection efficiency is determined from the luciferase activity in cells measured by luminescence. ODN corresponds to cytosolic and nuclear transfer of fluorescein-labelled oligonucleotide in the presence of histidylated oligolysine, evaluated under confocal microscope. Cytotoxicity was evaluated by using the colorimetric MTT assay. (MTT = 3-(4,5-dimethylthiazol-2-yl)-2,5-diphenyltetrazolium bromide)

## Claims

1. Oligomeric conjugate positively charged, containing an oligomer with a polymerization degree (PD) from 5 to 50, preferably 10 to 40 and more preferably 20, formed from monomeric components having free NH₃⁺ in a number equal to or higher than 50 % of the polymerization degree,
said oligomer being as follows:
- the free NH₃⁺ of the above-mentioned components are substituted in a ratio of at least 50 %, advantageously from 60 % to 95 %, particularly 80 to 90 % (this ratio being determined by nuclear magnetic resonance), by protonable residues in a weak acid medium, the pH of which is lower than 7.4, leading in such a weak acid medium to a destabilization of cellular membranes,
- the above-mentioned protonable residues possess in addition the following properties:
→ they contain a functional group enabling them to be linked to the above-mentioned oligomer,
→ they do not correspond to a recognition signal recognized by a cellular membrane receptor,
→ they can comprise at least one free NH₃⁺ group,
- the free NH₃⁺ of the above-mentioned monomers can be also substituted by an uncharged residues leading to a reduction of the number of positive charges in comparison to the same oligomeric before substitution,
- molecules constituting a recognition signal recognized by a membrane cellular receptor may be present:
→ either by substitution of some of the free NH₃⁺ of the above-mentioned monomers,
→ either on some of the uncharged residues leading to a reduction of the number of charges,
→ either on some of the above-mentioned protonable residues leading to a destabilization of the cellular membranes,
→ or by substitution of the free NH₃⁺ (if it is present) of the above-mentioned protonable residues leading to a destabilization of the cellular membrane,
provided that:
1) the total number of the non substituted NH₃⁺ functions is of at least 50 % of the polymerization degree,
2) the number of monomers initially carrying free NH₃⁺ is substituted in a ratio of at least 50 % of the polymerization degree by residues leading to a destabilization of the cellular membrane.

2. Oligomeric conjugate according to claim 1, wherein the protonable residues leading to a destabilization of cellular membranes, present the additional properties:
- they are weak bases the pK of which in aqueous medium is lower than 8, so that a proportion higher than 50% of these bases linked to a cationic oligomer is not protonated in a neutral medium of pH 7.4.

3. Oligomeric conjugate complex according to claim 1, wherein the protonable residues leading to a destabilization of cellular membranes, present the additional properties:
- they belong to the group of compounds comprising an imidazole ring,
- they belong to the group of quinolins,
- they belong to the group ofpterins,
- they belong to the group of pyridins.

4. Oligomeric conjugate according to anyone of claims 1 to 3, wherein the protonable residues leading to a destabilization of the cellular membranes are:
- alkylimidazoles in which the alkyl radical comprises from 1 to 10, particularly from 2 to 6 carbon atoms, and in which only one of the nitrogen atoms of the imidazole ring is substituted.

5. Oligomeric conjugate according to anyone of claims 1 to 4, wherein the protonable residues leading to a destabilization of cellular membranes are chosen from:
histidine, 4-carboxymethyl-imidazole,
3-(1-methyl-imidazol-4yl)-alanine, 3-(3-methyl-imidazol-4yl)-alanine,
2-carboxy-imidazole, histamine, 3-imidazol-4yl)-L-lactic acid,
2-(1-methyl-imidazol-4yl)ethylamine, 2-(3-metyl-limidazol-4yl)ethylamine,
β-alanyl-histidine-(carnosine), 7-chloro-4(amino-1-methylbutylamino)-quinoline,
N⁴-(7-chloro-4-quinolinyl)-1,4-pentanediamine,
8-(4-amino-1-methylbutylamino)-6-methoxy-quinoline (primaquine),
N⁴-(6-methoxy-8-quinolinyl)1,4-pentanediamine, quininic acid,
quinoline carboxylic acid, pteroïc acid, nicotinic acid, quinolinic acid.

6. Oligomeric conjugate according to anyone of claims 1 to 5, wherein the oligomeric conjugate contains an oligomer of the following formula: 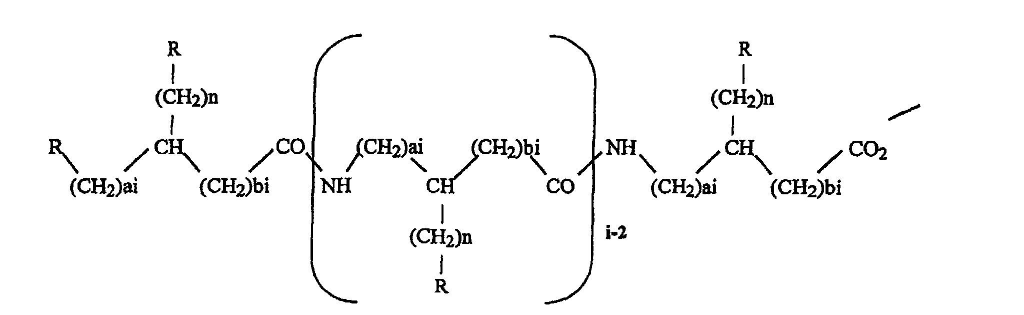 wherein
* ai is an integer varying from 0 to 10,
* bi is an integer varying from 0 to 10,
* i = degree of polymerization from 5 to 50, and particularly 10 to 40, and preferably 20,
* n is an integer varying from 1 to 6, and preferably 4,
* R represents in a ratio of 50 % to 100 % (corresponding to a number u) 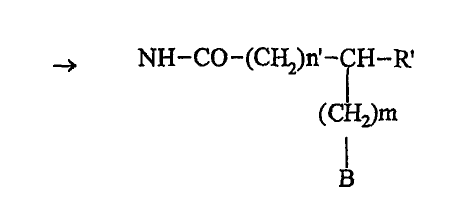 or 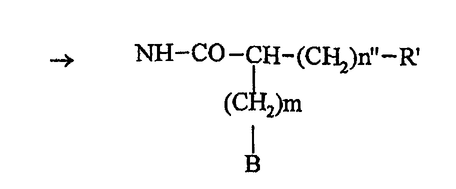 or 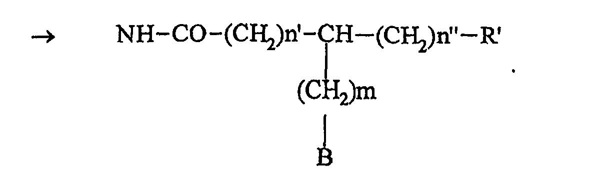
m is an integer varying from 1 to 6,
n' is an integer varying from 0 to 6,
n" is an integer varying from 0 to 6,
B is a weak base as defined according to anyone of claims 2 to 4,
R' represents NH₃⁺ (corresponding to a number p),
or NH (corresponding to a number q) substituted by
-CO-CH₃
- CO-(CHOH)rH r being an integer from 1 to 15, and preferably 1 to 7
- CO-(CH₂)s-(CHOH)rH r being an integer from 1 to 15, and preferably 1 to 7, and s being an integer from 1 to 6, and preferably 6 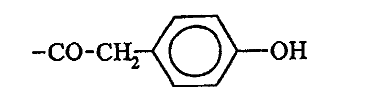 -SO₂-Flu
- CO-Flu
-CS-NH-Flu
Flu being a fluorescent molecule
* R represents in a ratio of 0 % to 50 % (corresponding to f: 0 < f ≤ u)
- NH₃⁺ (corresponding to a number j),
- NH (corresponding to a number k), substituted by
-CO-CH₃
- CO-(CHOH)rH r being an integer from 1 to 15, and preferably 1 to 7
- CO-(CH₂)s-(CHOH)rH r being an integer from 1 to 15, and preferably 1 to 7, and s being an integer from 1 to 6, and preferably 6 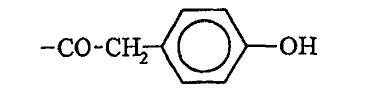 -SO₂-Flu
- CO-Flu
- CS-NH-Flu
Flu being a fluorescent molecule
- H (corresponding to a number h)
- (CH₂) nH, n being an integer from 1 to 6 (corresponding to a number h)
- (CH₂) n-OH n being an integer from 1 to 6 (corresponding to a number h)
- (CH₂) n-SA' A' = H, CH₃ or 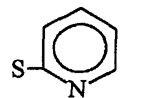 (corresponding to a number h) n being integer from 1 to 6
with
. i = u + j + k + h
. total number of α NH₃⁺ = p = u-q
. total number of ω NH₃⁺ = j = f-(k + h)
. total number of NH₃⁺ = m = p + j+1
with the proviso that:
1) u ≥ i/2
2)m≥i/2

7. Oligomeric conjugate according to anyone of claims 1 to 6, wherein the oligomeric conjugate contains an oligomer of the following formula: 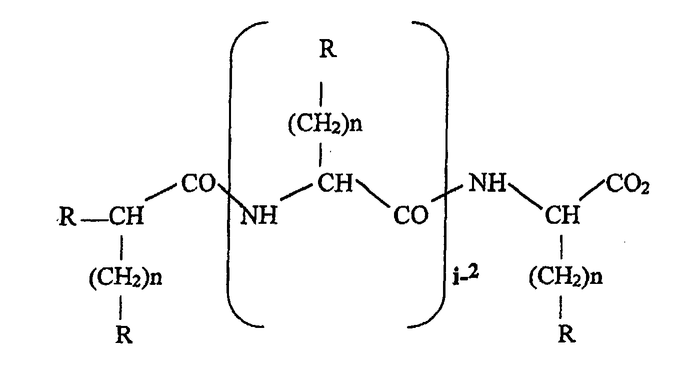 wherein
* i = degree of polymerization from 5 to 50, and particularly 10 to 40, and preferably 20,
* n is an integer varying from 1 to 6, and preferably 4,
* R represents in a ratio of 50 % to 100 % (corresponding to u) 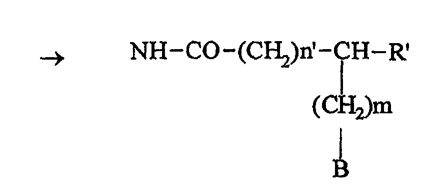 or 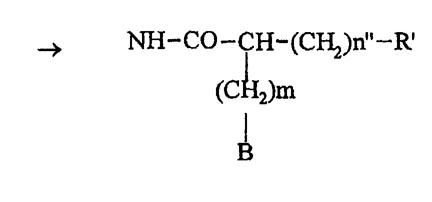 or 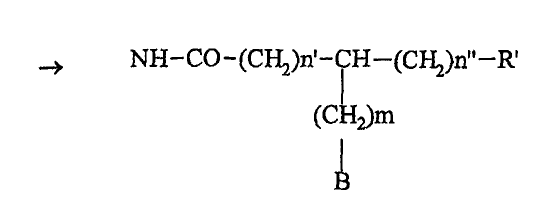
m is an integer varying from 1 to 6,
n' is an integer varying from 0 to 6, .
n" is an integer varying from 0 to 6,
B is a weak base as defined according to anyone of claims 2 to 4,
R' represents NH₃⁺ (corresponding to a number p),
or NH (corresponding to a number q) substituted by
-CO-CH₃
- CO-(CHOH)rH r being an integer from 1 to 15, and preferably 1 to 7
- CO-(CH₂)s-(CHOH)rH r being an integer from 1 to 15, and preferably 1 to 7, and s being an integer from 1 to 6, and preferably 6 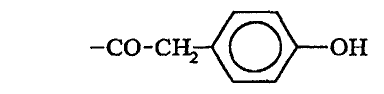 -SO₂-Flu
- CO-Flu
- CS-NH-Flu
Flu being a fluorescent molecule
* R represents in a ratio of 0% to 50% (corresponding to f: 0<f≤1)
- NH₃⁺ (corresponding to a number j),
- NH (corresponding to a number k), substituted by
-CO-CH₃
- CO-(CHOH)rH r being an integer from 1 to 15, and preferably 1 to 7
- CO-(CH₂)s-(CHOH)rH r being an integer from 1 to 15, and preferably 1 to 7, and s being an integer from 1 to 6, and preferably 6 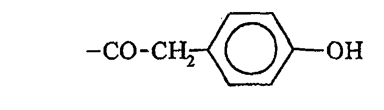 -SO₂-Flu
-CO-Flu
-CS-NH-Flu
Flu being a fluorescent molecule
- H (corresponding to a number h)
- (CH₂)nH, n being an integer from 1 to 6
(corresponding to a number h)
- (CH₂)ₙ-OH n being an integer from 1 to 6
(corresponding to a number h)
- (CH₂)ₙ-SA' A' = H, CH₃ or 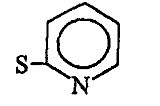 (corresponding to a number h) n being integer from 1 to 6
with
. i=u+j+k+h
. total number of α NH₃⁺ = p = u-q
. total number of ω NH₃⁺ = j = f-(k + h)
. total number of NH₃⁺ = m = p + j + 1
with the proviso that:
1)u ≥ i/2
2)m ≥ i/2

8. Oligomeric conjugate according to anyone of claims 1 to 7, wherein the oligomeric conjugate contains an oligomer of the formula according to claim 7, wherein
i = 19 n = 4 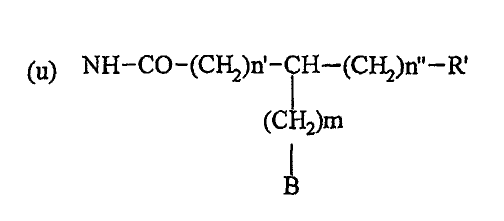 wherein
n' = n" = 0
R' = NH₃⁺
m = 1
B = imidazole
R = 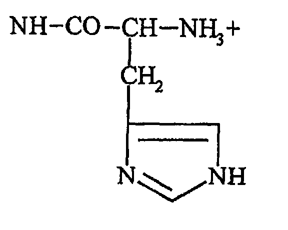
(f) R = NH⁺₃
u = 12
j = 7
or
i = 19 n = 4 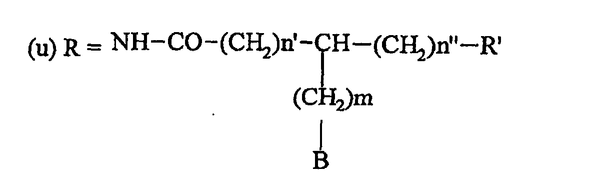 wherein
n' = n" = 0
R' = NH₃⁺
m = 1
B = imidazole
R = 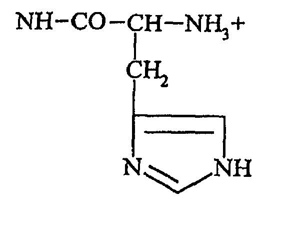
(f) R = NH₃⁺
u = 16
j = 3
i = 19 n = 4 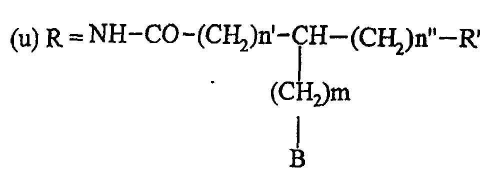 wherein
n' - n" = 0
R' = NH₃⁺
m = 1
B = imidazole
R = 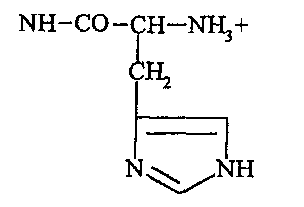
(f) R = NH₃⁺
u = 19
j = 0
or
i = 19 n = 4 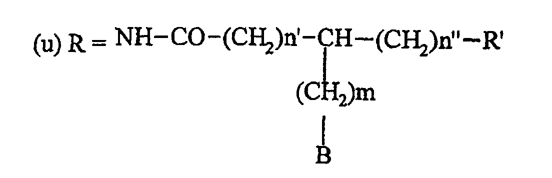 wherein
n' = n" = 0
R' = NH₃⁺
m = 1
B = imidazole
R = 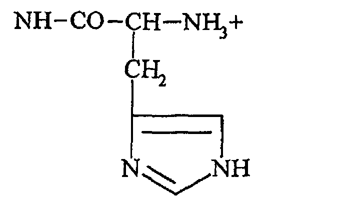
(f) R = CO-CH₃
u = 11
k = 8
or
i = 19 n = 4 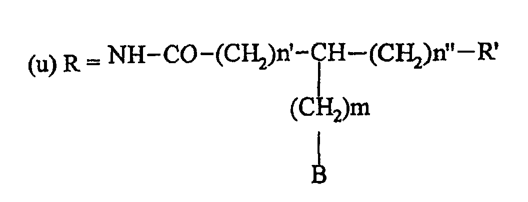 wherein
n' = n" = 0
R' = NH₃⁺
m = 1
B = imidazole
R = 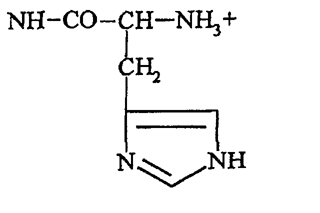
(f) R = CO-CH₃
u = 15
k = 4
or
i = 19 n = 4 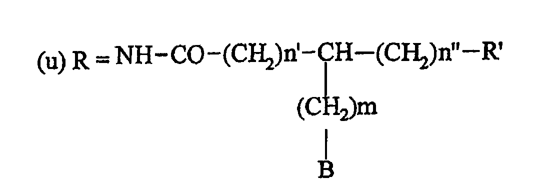 wherein
n' = n" = 0
R' = NH₃⁺
m = 1
B = imidazole
R = 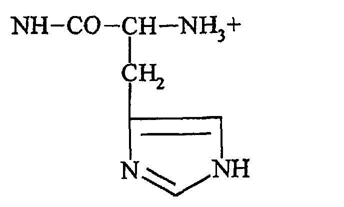
(f) R = CO-(CHOH)rH
r = 5
u = 12
k = 3
or
i = 19 n = 4 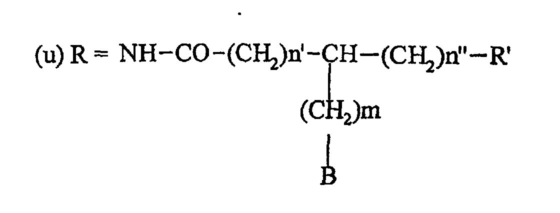 wherein
(q) n' = n" = 20
R' = NH-CO-CH₃
m = 1
B = imidazole
R = 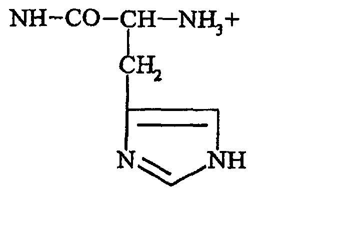
(f) R = NH₃⁺
u = 16
f = 4
k = 3

9. Composition containing at least one oligomeric conjugate according to anyone of claims 1 to 8, in association with at least a peptide, an oligoside or an oligonucleotide, or a mixture thereof.

10. Combined preparation containing as active substance the following individual components, in the form of a kit-of-parts:
- an oligomeric conjugate according to anyone of claims 1 to 8,
- at least one oligomeric conjugate according to anyone of claims 1 to 8, in association with at least a peptide, an oligoside or an oligonucleotide, or a mixture thereof,
for the simultaneous, separate or sequential use, for the *in vitro,* the *in vivo* or the *ex vivo* transfer of said biological molecules into the cytosol and/or cell nucleus.

11. Use of an oligomeric conjugate according to anyone of claims 1 to 8, for the preparation of a drug intended for the *in vitro,* the *ex vivo* or the *in vivo* intracellular transfer of a peptide, an oligoside or an oligonucleotide, into the cytosol and/or in the cell nucleus.

12. Use of an oligomeric conjugate according to anyone of claims 1 to 8 or of a composition according to claim 9, or of a combined preparation according to claim 10, for the preparation of a drug intended for the *in vitro,* the *ex vivo* or the *in vivo* intracellular transfer of a peptide, an oligoside or an oligonucleotide, or a mixture thereof, into the cytosol or/and in the cell nucleus.

13. Use according to claim 11 or 12, wherein the cells are chosen among muscular, epithelial, endothelial, myeloid cells such as monocytes, macrophages and fibroblasts, leukocytes and granulocytes, osteoblasts as well as dendritic cells, stem cells which are not of human embryonic origin, neuronal cells, or dermal cells.

14. Method for the *in vitro* or the *ex vivo* transfer of an oligonucleotide, wherein an oligonucleotide and an oligomeric conjugate according to anyone of claims 1 to 8 or of a composition according to claim 9, or of a combined preparation according to claim 10, are(is) contacted with a medium containing cells to be transferred, under conditions such that there is:
- transfer of an antisense oligonucleotide in the cytosol and/or the cell nucleus where it binds and blocks the complementary mRNA sequence,
- or transfer of an oligonucleotide as activator into the cytosol where it depresses or activates a second messenger in the cytosol, or the corresponding gene in the nucleus,
- or transfer into the cytosol and/or the cell nucleus of oligonucleotides corresponding to a repetitive bacterial type DNA sequence with stimulating or immunodepressive activity,
- or transfer of an oligonucleotide in the cell nucleus where it binds to DNA and forms a triple helix leading to the inhibition of gene expression,
- or transfer into the cytosol and/or the cell nucleus of RNA oligonucleotide acting as decoys which inhibit gene expression by blocking the binding of regulatory factors to the authentic DNA region,
- or transfer into the cytosol and/or the cell nucleus of ribozymes (RNA oligonucleotides) which inhibit gene expression by cleaving the mRNA.

15. Method for the *in vitro* or the *ex vivo* transfer of peptide, wherein a peptide and an oligomeric conjugate according to anyone of claims 1 to 8 or of a composition according to claim 9, or of a combined preparation according to claim 10, in particular wherein the peptide is an antigenic peptide, are(is) contacted with a medium containing cells to be transferred, under conditions such that there is a transfer of said antigenic peptide in the cytosol of antigen presenting cells (macrophages, dendritic cells and B cells) where they are processed in proteosomes in order to bind to MHCI molecules, allowing the presentation of the antigenic epitope fixed on MHCI.

16. Method for the *in vitro* or the *ex vivo* transfer of an oligoside, wherein an oligoside and an oligomeric conjugate according to anyone of claims 1 to 8 or of a composition according to claim 9, or of a combined preparation according to claim 10, are(is) contacted with a medium containing cells to be transferred, under conditions such that there is a transfer of said oligoside into the cytosol and/or the cell nucleus.

17. Pharmaceutical composition, comprising as active substance a composition according to claim 9, or a combined preparation according to claim 10, in association with a pharmaceutically acceptable vehicle.

18. Use of a composition according to claim 9, or of a combined preparation according to claim 10, for the preparation of a drug for use in the treatment of cancer, inflammatory or immunology diseases (such as graft rejection, allergy, auto-immunity) or infectious diseases.

19. Kit or case containing:
- an oligomeric conjugate according to anyone of claims 1 to 8, substituted by a protonable residue leading in a weak acid medium, the pH of which is lower than 7.4, to a destabilization of cellular membranes, this oligomeric conjugate being able to comprise a recognition signal, which is previously fixed or not on the above-said conjugate, said recognition signal being dependent upon the cell to target,
- at least one peptide, one oligoside or one oligonucleotide to transfer,
- optionally reagents enabling the possible binding of the recognition signal on the above-said oligomeric conjugate,
- optionally reagents enabling the formation of a composition according to claim 9, or of a combined preparation according to claim 10,
- reagents enabling the transfer of the peptide, the oligoside or the oligonucleotide in the cytosol and/or the cell nucleus.

## Patentansprüche

1. Positiv geladenes, oligomeres Konjugat, das ein Oligomer mit einem Polymerisationsgrad (PG) von 5 bis 50, bevorzugt 10 bis 40 und mehr bevorzugt 20 enthält, gebildet von monomeren Bestandteilen mit freien NH₃⁺ in einer Anzahl größer oder gleich 50% des Polymerisationsgrades,
wobei das Oligomer wie folgt ist:
- die freien NH₃⁺ der vorher erwähnten Bestandteile sind in einem Verhältnis von 50%, vorteilhafterweise von 60% bis 95%, insbesondere von 80 bis 90% substituiert (dieses Verhältnis wird durch magnetische Kernresonanz bestimmt) durch Reste, die in einem schwach sauren Medium, dessen pH niedriger als 7,4 ist, protoniert werden können, was in einem derartig schwach sauren Medium zu einer Destabilisierung von Zellmembranen führt,
- wobei die vorher erwähnten Reste, die protoniert werden können, zusätzlich die folgenden Eigenschaften besitzen:
→ sie besitzen eine funktionelle Gruppe, welche sie in die Lage versetzt, an das vorher erwähnte Oligomer gebunden zu werden,
→ sie entsprechen nicht einem durch einen zellulären Membranrezeptor erkannten Erkennungssignal,
→ sie können wenigstens eine freie NH₃⁺-Gruppe umfassen,
- wobei die freien NH₃⁺ der vorher erwähnten Monomere ebenfalls durch einen ungeladen Rest substituiert sein können, was zu einer Reduktion der Anzahl der positiven Ladungen im Vergleich zu dem gleichen Oligomer vor der Substitution führt,
- wobei die Moleküle, die ein durch einen zellulären Membranrezeptor erkanntes Erkennungssignal bilden, vorhanden sein können:
→ entweder durch Substitution einiger der freien NH₃⁺ der vorher erwähnten Monomere,
→ oder von einigen der ungeladenen Reste, was zu einer Reduktion der Anzahl der Ladungen führt,
→ oder von einigen der vorher erwähnten Reste, die protoniert werden können, was zu einer Destabilisierung der Zellmembranen führt,
→ oder durch Substitution des freien NH₃⁺ (wenn es vorhanden ist) der vorher erwähnten Reste, die protoniert werden können, was zu einer Destabilisierung der Zellmembran führt,
vorausgesetzt, dass
1) die Gesamtzahl der nicht substituierten NH₃⁺-Funktionen wenigstens 50% des Polymerisationsgrades ist,
2) die Anzahl der anfänglich freies NH₃⁺ tragenden Monomere in einem Verhältnis von wenigsten 50% des Polymerisationsgrades durch Reste substituiert wird, die zu einer Destabilisierung der Zellmembran führen.

2. Oligomeres Konjugat nach Anspruch 1, wobei die Reste, die protoniert werden können, die zu einer Destabilisierung der Zellmembranen führen, die zusätzlichen Eigenschaften aufweisen:
- sie sind schwache Basen, deren pK in wässrigem Medium niedriger als 8 ist, so dass ein Anteil von mehr als 50% von diesen Basen gebunden an ein kationisches Oligomer in einem neutralen Medium mit pH 7,4 nicht protoniert wird.

3. Oligomeres Konjugat nach Anspruch 1, wobei die Reste, die protoniert werden können, die zu einer Destabilisierung der Zellmembranen führen, die zusätzlichen Eigenschaften aufweisen:
- sie gehören zu der Gruppe von Verbindungen mit einem Imidazolring,
- sie gehören zu der Gruppe der Chinoline,
- sie gehören zu der Gruppe der Pterine,
- sie gehören zu der Gruppe der Pyridine.

4. Oligomeres Konjugat nach einem der Ansprüche 1 bis 3, wobei die Reste, die protoniert werden können, die zu einer Destabilisierung der Zellmembranen führen, sind:
- Alkylimidazole, in denen das Alkylradikal von 1 bis 10, insbesondere von 2 bis 6 Kohlenstoffatome umfasst, und in denen nur eins der Stickstoffatome des Imidazolrings substituiert ist.

5. Oligomeres Konjugat nach einem der Ansprüche 1 bis 4, wobei die Reste, die protoniert werden können, die zu einer Destabilisierung der Zellmembran führen, ausgewählt werden aus:
Histidin, 4-Carboxymethyl-imidazol, 3-(1-Metllyl-imidazol-4yl)-alanin, 3-(3-Methyl-imidazol-4yl)-alanin, 2-Carboxy-imidazol, Histamin, 3-Imidazol-4yl-L-Milchsäure, 2-(1-Methyl-imidazol-4yl)ethylamin, 2-(3-Methyl-imidazol-4yl)ethylamin, β-Alanyl-Histidin-(Carnosin), 7-Chlor-4(Amino-1-Methylbutylamino)-chinolin, N⁴-(7-Chlor-4-chinolinyl)-1,4-pentandiamin, 8-(4-Amino-1-methylbutylamino)-6-methoxychinolin (Primachin), N⁴-(6-Methoxy-8-chinolinyl)-1,4-pentandiamin, Chininsäure, Chinolincarbonsäure, Pteroinsäure, Nikotinsäure, Chinolinsäure.

6. Oligomeres Konjugat nach einem der Ansprüche 1 bis 5, wobei das oligomere Konjugat ein Oligomer der folgenden Formel enthält: 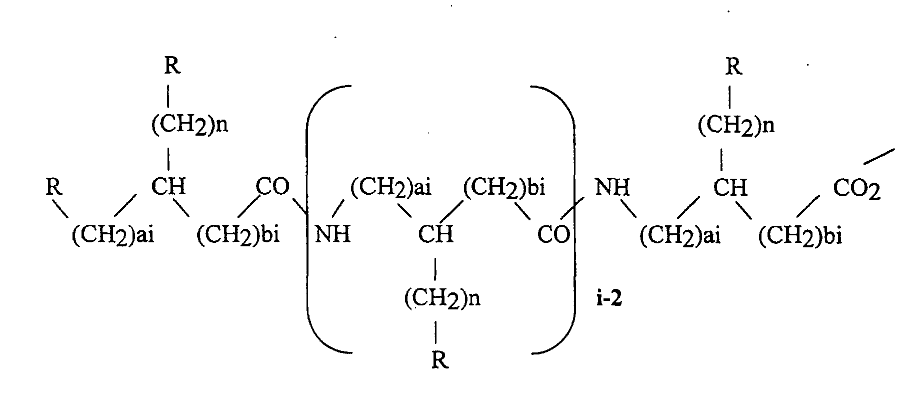 wobei
* ai eine ganze Zahl, variierend von 0 bis 10 ist,
* bi eine ganze Zahl, variierend von 0 bis 10 ist,
* i = der Polymerisationsgrad von 5 bis 50, und insbesondere 10 bis 40, und bevorzugt 20 ist,
* n eine ganze Zahl, variierend von 1 bis 6 und bevorzugt 4 ist,
* R stellt in einem Verhältnis von 50% bis 100% dar (entsprechend zu einer Anzahl u) 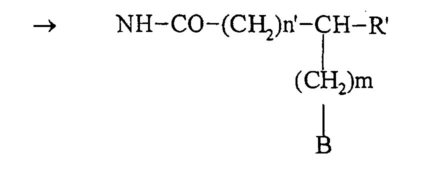 oder 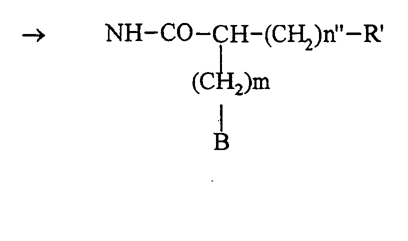 oder 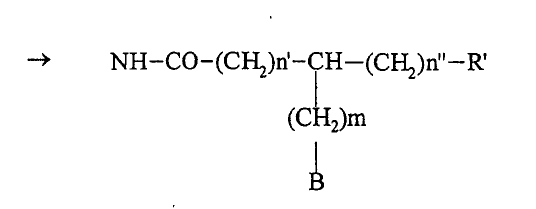
m eine ganze Zahl, variierend von 1 bis 6 ist,
n' eine ganze Zahl, variierend von 0 bis 6 ist,
n" eine ganze Zahl, variierend von 0 bis 6 ist,
B eine schwache Base, nach einem der Ansprüche 2 bis 4 ist,
R' NH₃⁺ (entsprechend einer Anzahl p),
oder NH (entsprechend einer Anzahl q) darstellt, substituiert durch
-CO-CH₃
- CO-(CHOH)rH wobei r eine ganze Zahl von 1 bis 15, und bevorzugt 1 bis 7 ist
-CO-(CH₂)s-(CHOH)rH wobei r eine ganze Zahl von 1 bis 15, und bevorzugt 1 bis 7, und s eine ganze Zahl von 1 bis 6, und bevorzugt 6 ist, 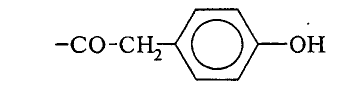 -SO₂-Flu
- CO-Flu
- CS-NH-Flu
wobei Flu ein fluoreszierendes Molekül ist
* R stellt in einem Verhältnis von 0% bis 50% dar (entsprechend zu f: 0 <f≤u)
- NH₃⁺ (entsprechend zu einer Anzahl j),
- NH (entsprechend zu einer Anzahl k), substituiert durch
-CO-CH₃
- CO-(CHOH)rH wobei r eine ganze Zahl von 1 bis 15, und bevorzugt 1 bis 7 ist
- CO-(CH₂)s-(CHOH)rH wobei r eine ganze Zahl von 1 bis 15, und bevorzugt 1 bis 7, und s eine ganze Zahl von 1 bis 6, und bevorzugt 6 ist, 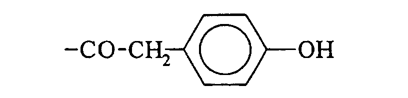 -SO₂-Flu
- CO-Flu
-CS-NH-Flu
wobei Flu ein fluoreszierendes Molekül ist
- H (entsprechend zu einer Anzahl h)
- (CH₂)nH n eine ganze Zahl von 1 bis 6 ist
(entsprechend zu einer Anzahl h),
- (CH₂)n-OH n eine ganze Zahl von 1 bis 6 ist
(entsprechend zu einer Anzahl h)
- (CH₂) n-SA' A' = H, CH₃ oder 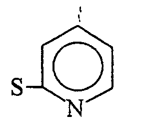 (entsprechend zu einer Anzahl h) n eine ganze Zahl von 1 bis 6 ist
mit
. i=u+j+k+h
. Gesamtanzahl von αNH₃⁺ = p = u - q
. Gesamtanzahl von ωNH₃⁺ = j = f - (k + h)
. Gesamtanzahl von NH₃⁺ = m = p + j + 1
unter der Bedingung, dass
1 ) u ≥ i/2
2) m ≥ i/2.

7. Oligomeres Konjugat nach einem der Ansprüche 1 bis 6, wobei das oligomere Konjugat ein Oligomer der folgenden Formel enthält: 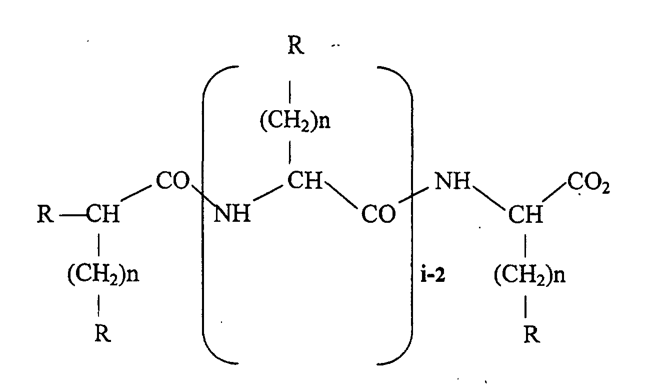 wobei
* i = der Polymerisationgrad von 5 bis 50 und insbesondere 10 bis 40 und bevorzugt 20 ist,
* n eine ganze Zahl, variierend von 1 bis 6 und bevorzugt 4 ist,
* R stellt in einem Verhältnis von 50% bis 100% dar (entsprechend zu einer Anzahl u) 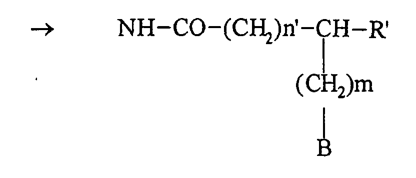 oder 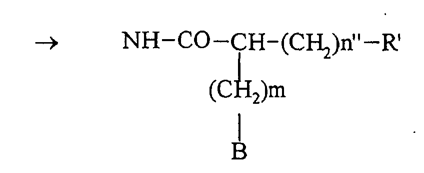 oder 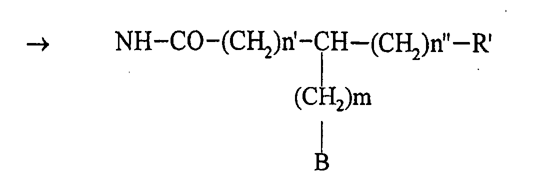
m eine ganze Zahl, variierend von 1 bis 6 ist,
n' eine ganze Zahl, variierend von 0 bis 6 ist,
n" eine ganze Zahl, variierend von 0 bis 6 ist,
B eine schwache Base, nach einem der Ansprüche 2 bis 4 ist,
R' NH₃⁺ (entsprechend einer Anzahl p),
oder NH (entsprechend einer Anzahl q) darstellt, substituiert durch
-CO-CH₃
- CO-(CHOH)rH wobei r eine ganze Zahl von 1 bis 15, und bevorzugt 1 bis 7 ist
- CO-(CH₂)s-(CHOH)rH wobei r eine ganze Zahl von 1 bis 15, und bevorzugt 1 bis 7, und s eine ganze Zahl von 1 bis 6, und bevorzugt 6 ist, 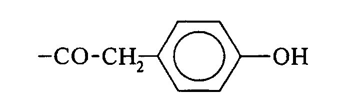 -SO₂-Flu
- CO-Flu
- CS-NH-Flu
wobei Flu ein fluoreszierendes Molekül ist
* R stellt in einem Verhältnis von 0% bis 50% dar (entsprechend zu f:0<f≤ 1)
- NH₃⁺ (entsprechend zu einer Anzahl j),
- NH (entsprechend zu einer Anzahl k), substituiert durch
-CO-CH₃
- CO-(CHOH)rH wobei r eine ganze Zahl von 1 bis 15, und bevorzugt 1 bis 7 ist
- CO-(CH₂)s-(CHOH)rH wobei r eine ganze Zahl von 1 bis 15, und bevorzugt 1 bis 7, und s eine ganze Zahl von 1 bis 6, und bevorzugt 6 ist, 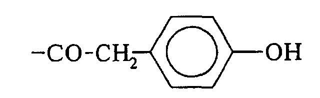 -SO₂-Flu
- CO-Flu
- CS-NH-Flu
wobei Flu ein fluoreszierendes Molekül ist
- H (entsprechend zu einer Anzahl h)
- (CH₂)nH n eine ganze Zahl von 1 bis 6 ist
(entsprechend zu einer Anzahl h)
- (CH₂)n-OH n eine ganze Zahl von 1 bis 6 ist
(entsprechend zu einer Anzahl h)
- (CH₂)ₙ-SA' A' = H, CH₃ oder 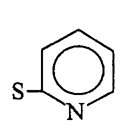 (entsprechend zu einer Anzahl h) n eine ganze Zahl von 1 bis 6 ist
mit
. i=u+j+k+h
. Gesamtanzahl von αNH₃⁺ = p = u - q
. Gesamtanzahl von ωNH₃⁺ = j = f - (k + h)
. Gesamtanzahl von NH₃⁺ = m = p + j + 1
unter der Bedingung, dass
1) u ≥ i/2
2) m ≥ i/2.

8. Oligomeres Konjugat nach einem der Ansprüche 1 bis 7, wobei das oligomere Konjugat ein Oligomer der Formel nach Anspruch 7 enthält, wobei
i = 19 n = 4 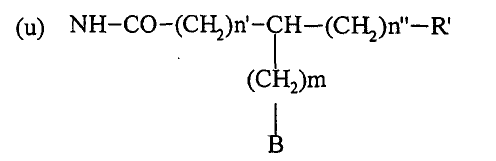 wobei
n' = n" = 0
R' = NH₃⁺
m = 1
B = Imidazol
R = 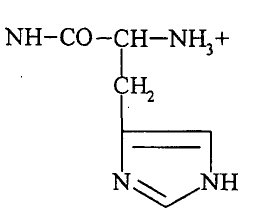
(f) R = NH⁺₃
u = 12
j = 7
oder
i = 19 n = 4 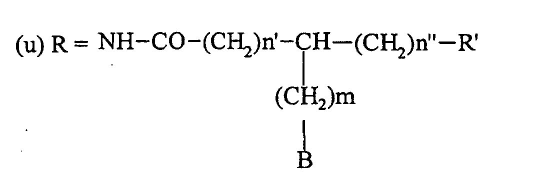 wobei
n' = n"=0
R' = NH₃⁺
m = 1
B = Imidazol
R = 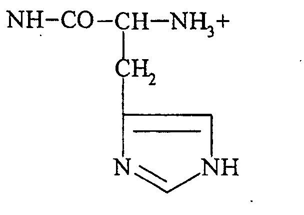
(f) R = NH₃⁺
u = 16
j = 3
oder
i = 19 n = 4 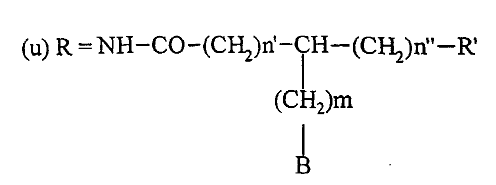 wobei
n' = n" = 0
R' = NH₃⁺
m = 1
B = Imidazol
R = 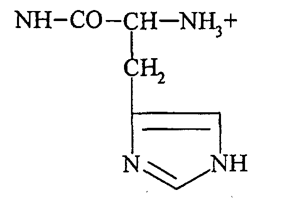
(f) R = NH₃⁺
u = 19
j = 0
oder
i = 19 n = 4 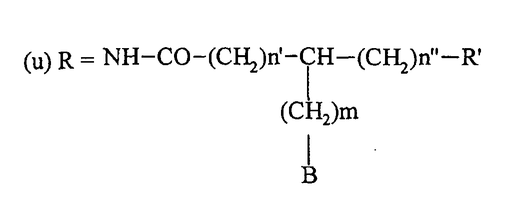 wobei
n' = n" = 0
R' = NH₃⁺
m = 1
B = Imidazol
R = 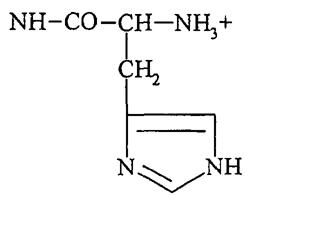
(f) R = CO-CH₃
u = 11
k = 8
oder
i = 19 n = 4 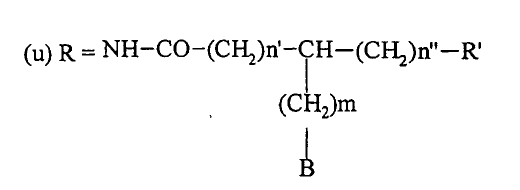 wobei
n' = n"= 0
R' = NH₃⁺
m = 1
B = Imidazol
R = 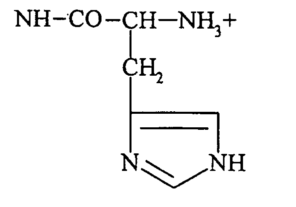
(f) R = CO-CH₃
u = 15
k = 4
oder
i = 19 n = 4 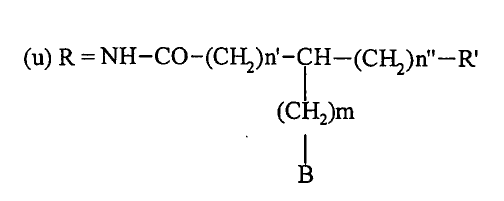 wobei
n' = n" = 0
R' = NH₃⁺
m = 1
B = Imidazol
R = 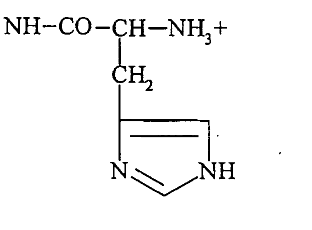
(f) R = CO-(CHOH)rH
r = 5
u = 12
k = 3
oder
i = 19 n = 4 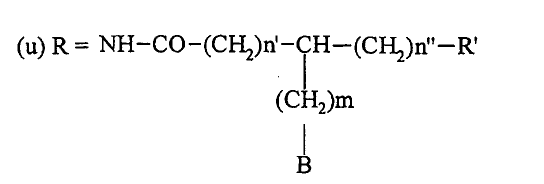 wobei
(q) n' = n" = 20
R' = NH-CO-CH₃
m = 1
B = Imidazol
R = 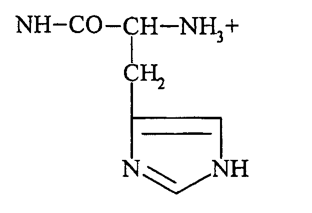
(f) R = NH₃⁺
u = 16
f = 4
k = 3

9. Zusammensetzung, die wenigstens ein oligomeres Konjugat nach einem der Ansprüche 1 bis 8 enthält, in Verbindung mit wenigstens einem Peptid, einem Oligosaccharid oder einem Oligonukeotid, oder einer Mischung davon.

10. Kombinierte Zubereitung, die als einen Wirkstoff die folgenden Einzelbestandteile in der Form einer Kollektion (kit-of-parts) enthält:
- ein oligomeres Konjugat nach einem der Ansprüche 1 bis 8,
- wenigstens ein oligomeres Konjugat nach einem der Ansprüche 1 bis 8, in Verbindung mit wenigstens einem Peptid, einem Oligosaccharid oder einem Oligonukeotid, oder einer Mischung davon,
für die gleichzeitige, getrennte oder aufeinanderfolgende Verwendung, für den *in vitro*-, den *in vivo*- oder den *ex vivo-*Transfer des biologischen Moleküls in das Cytosol und/oder den Zellkern.

11. Verwendung eines oligomeren Konjugats nach einem der Ansprüche 1 bis 8 für die Herstellung eines Arzneimittels bestimmt für den intrazellulären *in vitro-,* den *ex vivo*- oder den *in vivo-* Transfer eines Peptids, eines Oligosaccharids oder eines Oligonukleotids in das Cytosol und/oder den Zellkern.

12. Verwendung eines oligomeren Konjugats nach einem der Ansprüche 1 bis 8 oder einer Zusammensetzung nach Anspruch 9 oder einer kombinierten Zubereitung nach Anspruch 10, für die Herstellung eines Arzneimittels bestimmt für den intrazellulären *in vitro-,* den *ex vivo-* oder den *in vivo-* Transfer eines Peptids, eines Oligosaccharids oder eines Oligonukleotids in das Cytosol und/oder den Zellkern.

13. Verwendung nach Anspruch 11 oder 12, wobei die Zellen ausgewählt sind aus muskulären, epithelialen, endothelialen, myeloiden Zellen, wie etwa Monocyten, Makrophagen, und Fibroblasten, Leukocyten und Granulocyten, Osteoblasten als auch dendritischen Zellen, Stammzellen, welche nicht humanen embryonalen Ursprungs sind, neuronalen Zellen oder Hautzellen.

14. Verfahren für den *in vitro-* oder den *ex vivo*-Transfer eines Oligonukleotids, wobei ein Oligonukleotid und ein oligomeres Konjugat nach einem der Ansprüche 1 bis 8 oder eine Zusammensetzung nach Anspruch 9, oder eine kombinierte Zubereitung nach Anspruch 10, in Kontakt mit dem Medium der zu transferierenden Zellen gebracht werden (wird), unter derartigen Bedingungen, dass:
- ein Transfer eines Antisense-Oligonukleotids in das Cytosol und/oder den Zellkern stattfindet, wo es die komplementäre mRNA-Sequenz bindet und blockiert,
- oder ein Transfer eines Oligonukleotids als ein Aktivator in das Cytosol stattfindet, wo er einen zweiten Botenstoff oder das entsprechende Gen in dem Kern deprimiert oder aktiviert,
- oder ein Transfer in das Cytosol und/oder den Zellkern von Oligonukleotiden stattfindet, die einer repetitiven bakteriellen DNA-Sequenz mit stimulierender oder immunosuppressiver Wirkung entsprechen,
- oder ein Transfer eines Oligonukleotids in den Zellkern stattfindet, wo es DNA bindet und eine Triplehelix bildet, was zur Hemmung der Genexpression führt,
- oder ein Transfer in das Cytosol und/oder den Zellkern von einem RNA-Oligonukleotid stattfindet, das als Köder wirkt, welcher die Genexpression durch Blockierung der Bindung der regulatorischen Faktoren des authentischen DNA-Bereichs hemmt,
- oder ein Transfer in das Cytosol und/oder den Zellkern von Ribozymen (RNA-Oligonukleotiden) stattfindet, welche die Genexpression durch Spaltung der mRNA hemmen.

15. Verfahren für den *in vitro-* oder den *ex vivo*-Transfer eines Peptids, wobei ein Peptid und ein oligomeres Konjugat nach einem der Ansprüche 1 bis 8 oder eine Zusammensetzung nach Anspruch 9 oder eine kombinierte Zubereitung nach Anspruch 10, insbesondere wobei das Peptid ein antigenes Peptid ist, in Kontakt mit dem Medium zu transferierender Zellen gebracht werden (wird), unter derartigen Bedingungen, dass ein Transfer des antigenen Peptids in das Cytosol von Antigen-präsentierenden Zellen (Makrophagen, dendritische Zellen, B-Zellen) stattfindet, wo sie in Proteosomen verarbeitet werden, um an MHCI-Moleküle zu binden, was die Präsentation des antigenen Epitops fixiert an MHCI ermöglicht.

16. Verfahren für den *in vitro-* oder den *ex vivo*-Transfer eines Oligosaccharids, wobei ein Oligosaccharid und ein oligomeres Konjugat nach einem der Ansprüche 1 bis 8, oder eine Zusammensetzung nach Anspruch 9, oder eine kombinierte Zubereitung nach Anspruch 10 in Kontakt mit dem Medium der zu transferierenden Zellen gebracht werden (wird), unter Bedingungen, dass dort ein Transfer des Oligosaccharids in das Cytosol und/oder den Zellkern stattfindet.

17. Pharmazeutische Zusammensetzung, die als Wirkstoff eine Zusammensetzung nach Anspruch 9, oder eine kombinierte Zubereitung nach Anspruch 10, in Verbindung mit einer pharmazeutisch akzeptablen Trägersubstanz, enthält.

18. Verwendung einer Zusammensetzung nach Anspruch 9 oder einer kombinierten Zubereitung nach Anspruch 10 für die Herstellung eines Arzneimittels zur Verwendung bei der Behandlung von Krebs, entzündlichen oder immunologischen Krankheiten (wie etwa Transplantatabstoßung, Allergie, Autoimmunität) oder Infektionskrankheiten.

19. Kit oder Behälter enthaltend:
- ein oligomeres Konjugat nach einem der Ansprüche 1 bis 8, substituiert durch einen Rest, der protoniert werden kann, was in einem schwach sauren Medium, wobei der pH niedriger als 7,4 ist, zu einer Destabilisierung zellulärer Membranen führt, wobei dieses oligomere Konjugat imstande ist ein Erkennungssignal zu umfassen, welches vorher an das oben erwähnte Konjugat fixiert wird oder nicht, wobei das Erkennungssignal abhängig von der Zielzelle ist,
- wenigstens ein Peptid, ein Oligosaccharid oder ein Oligonukleotid zum Transferieren,
- optionale Reagenzien, welche die mögliche Bindung des Erkennungssignals an das vorher erwähnte oligomere Konjugat ermöglichen,
- optionale Reagenzien, welche die Bildung einer Zusammensetzung nach Anspruch 9 oder einer kombinierten Zubereitung nach Anspruch 10 ermöglichen,
- Reagenzien, welche den Transfer des Peptids, des Oligosaccharids oder des Oligonukleotids in das Cytosol und/oder den Zellkern ermöglichen.

## Revendications

1. Conjugué oligomérique chargé positivement, contenant un oligomère avec un degré de polymérisation (DP) de 5 à 50, de préférence de 10 à 40 et préférentiellement de 20, formé à partir de composants monomériques possédant des NH₃⁺ libres dans un nombre égal à ou supérieur à 50 % du degré de polymérisation,
ledit oligomère étant comme suit :
- les NH₃⁺ libres des composants susmentionnés sont substitués dans un rapport d'au moins 50 %, avantageusement de 60 % à 95 %, notamment de 80 à 90 % (ce rapport étant déterminé par résonance magnétique nucléaire), par des résidus protonables dans un milieu faiblement acide, dont le pH est inférieur à 7,4, conduisant, dans un tel milieu faiblement acide, à une déstabilisation des membranes cellulaires,
- les résidus protonables susmentionnés possèdent en plus les propriétés suivantes :
→ ils contiennent un groupe fonctionnel leur permettant d'être lié à l'oligomère susmentionné,
→ ils ne correspondent pas à un signal de reconnaissance reconnu par un récepteur de membrane cellulaire,
→ ils peuvent comprendre au moins un groupe NH₃⁺ libre,
- les NH₃⁺ libres des monomères susmentionnés peuvent également être substitués par un résidu non chargé conduisant à une réduction du nombre de charges positives par rapport au même oligomère avant la substitution,
- des molécules constituant un signal de reconnaissance reconnu par un récepteur de membrane cellulaire peuvent également être présentes :
→ soit par substitution de certains des NH₃⁺ libres des monomères susmentionnés,
→ soit sur certains des résidus non chargés conduisant à une réduction du nombre de charges,
→ soit sur certains des résidus protonables susmentionnés conduisant à la déstabilisation des membranes cellulaires,
→ ou par substitution des NH₃⁺ libres (le cas échéant) des résidus protonables susmentionnés conduisant à une déstabilisation de la membrane cellulaire,
à condition que :
1) le nombre total des fonctions NH₃⁺ non substituées soit égal à au moins 50 % du degré de polymérisation,
2) le nombre de monomères portant initialement des NH₃⁺ libres est substitué dans un rapport d'au moins 50 % du degré de polymérisation par des résidus conduisant à une déstabilisation de la membrane cellulaire.

2. Conjugué oligomérique selon la revendication 1, dans lequel les résidus protonables conduisant à une déstabilisation des membranes cellulaires, présentent les propriétés supplémentaires suivantes :
- ils sont des bases faibles dont le pk en milieu aqueux est inférieur à 8, de telle sorte qu'une proportion supérieure à 50% de ces bases liées à un oligomère cationique n'est pas protonée dans un milieu neutre de pH 7,4.

3. Complexe conjugué oligomérique selon la revendication 1, dans lequel les résidus protonables conduisant à une déstabilisation des membranes cellulaires, présentent les propriétés supplémentaires suivantes :
- ils appartiennent au groupe de composés comprenant un noyau imidazole,
- ils appartiennent au groupe des quinolines,
- ils appartiennent au groupe des ptérines,
- ils appartiennent au groupe des pyridines.

4. Conjugué oligomérique selon l'une quelconque des revendications 1 à 3, dans lequel les résidus protonables conduisant à une déstabilisation des membranes cellulaires sont :
- des alkylimidazoles dans lesquels le radical alkyle comprend de 1 à 10, notamment de 2 à 6 atomes de carbone, et dans lesquels seulement un des atomes d'azote du noyau imidazole est substitué.

5. Conjugué oligomérique selon l'une quelconque des revendications 1 à 4, dans lequel les résidus protonables conduisant à une déstabilisation des membranes cellulaires sont choisis parmi :
histidine, 4-carboxyméthyl-imidazole,
3-(1-méthyl-imidazol-4yl)-alanine, 3-(3-méthyl-imidazol-4yl)-alanine,
2-carboxy-imidazole, histamine, acide 3-imidazol-4yl-L-lactique,
2-(1-méthyl-imidazol-4yl)éthylamine, 2-(3-méthyl-limidazol-4yl)éthylamine,
β-alanyl-histidine-(carnosine), 7-chloro-4(amino-1-méthylbutylamino)-quinoline,
N⁴-(7-chloro-4-quinolinyl)-1,4-pentanediamine,
8-(4-amino-1-méthylbutylamino)-6-méthoxy-quinoline (primaquine),
N⁴-(6-méthoxy-8-quinolinyl)-1,4-pentanediamine, acide quininique,
acide carboxylique de quinoline, acide ptéroïque, acide nicotinique,
acide quinolinique.

6. Conjugué oligomérique selon l'une quelconque des revendications 1 à 5, dans lequel le conjugué oligomérique contient un oligomère de formule suivante : 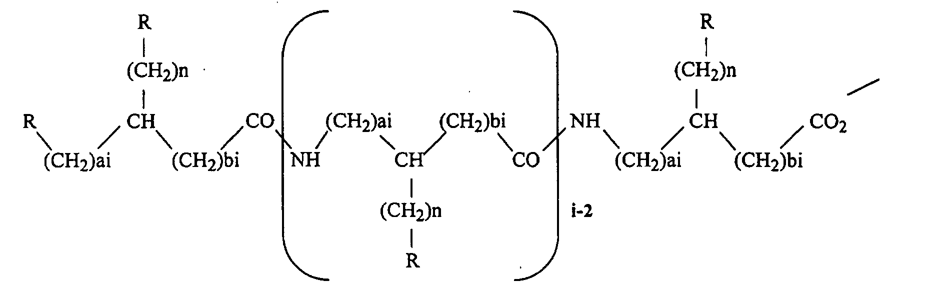 dans laquelle
* ai est un nombre entier variant de 0 à 10,
* bi est un nombre entier variant de 0 à 10,
* i = degré de polymérisation de 5 à 50, et notamment de 10 à 40, et de préférence égal à 20,
* n est un nombre entier variant de 1 à 6, et de préférence égal à 4,
* R représente dans un rapport de 50 % à 100 % (correspondant à un nombre u) 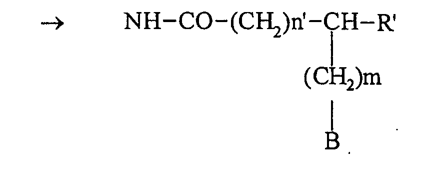
ou 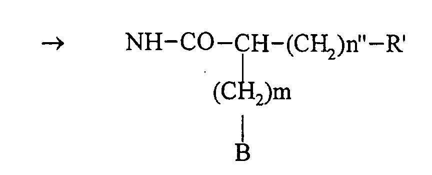 ou 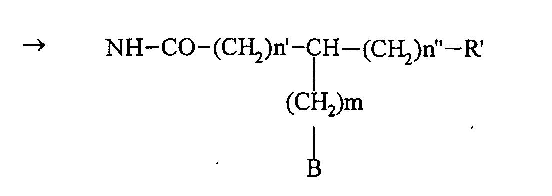
m est un nombre entier variant de 1 à 6,
n' est un nombre entier variant de 0 à 6,
n" est un nombre entier variant de 0 à 6,
B est une base faible telle que définie selon l'une quelconque des revendications 2 à 4,
R' représente NH₃⁺ (correspondant à un nombre p),
ou NH (correspondant à un nombre q) substitué par
-CO-CH₃
- CO-(CHOH)rH r étant un nombre entier variant de 1 à 15, et de préférence de 1 à 7
- CO-(CH₂)s-(CHOH)rH r étant un nombre entier variant de 1 à 15, et de préférence de 1 à 7, et s étant un nombre entier variant de 1 à 6, et de préférence égal à 6 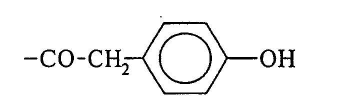 -SO₂-Flu
- CO-Flu
- CS-NH-Flu
Flu étant une molécule fluorescente
* R représente dans un rapport de 0 % à 50 % (correspondant à f : 0 < f ≤ u)
- NH₃⁺ (correspondant à un nombre j),
- NH (correspondant à un nombre k), substitué par
-CO-CH₃
- CO-(CHOH)rH r étant un nombre entier variant de 1 à 15, et de préférence de 1 à 7
- CO-(CH₂)s-(CHOH)rH r étant un nombre entier variant de 1 à 15, et de préférence de 1 à 7, et s étant un nombre entier variant de 1 à 6, et de préférence égal à 6 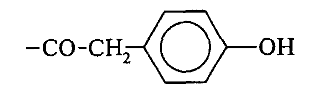 -SO₂-Flu
- CO-Flu
- CS-NH-Flu
Flu étant une molécule fluorescente
- H (correspondant à un nombre h)
- (CH₂) nH, n étant un nombre entier variant de 1 à 6
(correspondant à un nombre h)
- (CH₂) n-OH n étant un nombre entier variant de 1 à 6
(correspondant à un nombre h)
- (CH₂)n-SA' A' = H, CH₃ ou 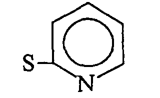
(correspondant à un nombre h) n étant un nombre entier variant de 1 à 6 avec
. i=u+j+k+h
. nombre total de α NH₃⁺ = p = u - q
. nombre total de ω NH₃⁺ = j = f - (k + h)
. nombre total de NH₃⁺ = m = p + j + 1
à condition que :
1) u ≥ i/2
2) m ≥ i/2

7. Conjugué oligomérique selon l'une quelconque des revendications 1 à 6, dans lequel le conjugué oligomérique contient un oligomère de formule suivante : 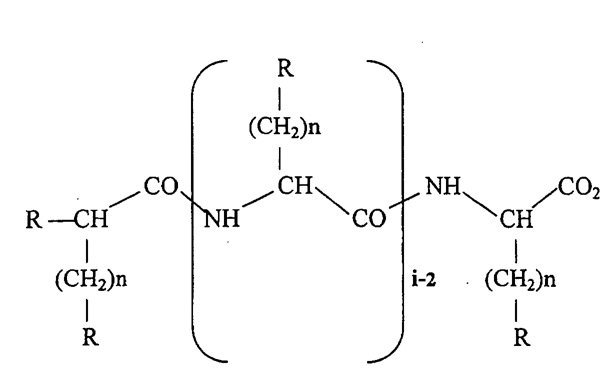 wherein
* i = degré de polymérisation variant de 5 à 50, et notamment de 10 à 40, et de préférence égal à 20,
* n est un nombre entier variant de 1 à 6, et de préférence égal à 4,
* R représente dans un rapport de 50 % à 100 % (correspondant à u) 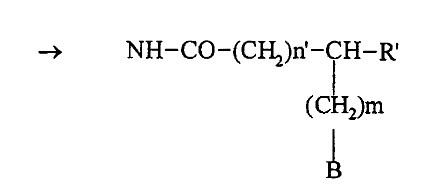
ou 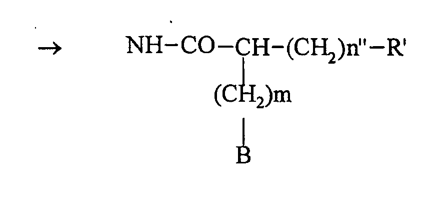 ou 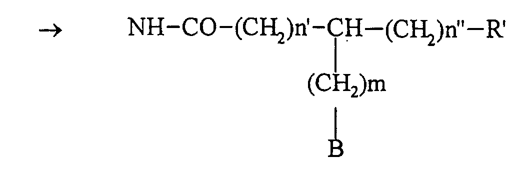
m est un nombre entier variant de 1 à 6,
n' est un nombre entier variant de 0 à 6,
n" est un nombre entier variant de 0 à 6,
B est une base faible telle que définie selon l'une quelconque des revendications 2 à 4,
R' représente NH₃⁺ (correspondant à un nombre p),
ou NH (correspondant à un nombre q) substitué par
-CO-CH₃
- CO-(CHOH)rH r étant un nombre entier variant de 1 à 15, et de préférence de 1 à 7
- CO-(CH₂)s-(CHOH)rH r étant un nombre entier variant de 1 à 15, et de préférence de 1 à 7, et s étant un nombre entier variant de 1 à 6, et de préférence égal à 6 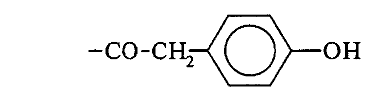 -SO₂-Flu
- CO-Flu
- CS-NH-Flu
Flu étant une molécule fluorescente
* R représente dans un rapport de 0 % à 50 % (correspondant à f:
0 < f ≤ 1)
- NH₃⁺ (correspondant à un nombre j),
- NH (correspondant à un nombre k), substitué par
-CO-CH₃
- CO-(CHOH)rH r étant un nombre entier variant de 1 à 15, et de préférence de 1 à 7
- CO-(CH₂)s-(CHOH)rH r étant un nombre entier variant de 1 à 15, et de préférence de 1 à 7 et s étant un nombre entier variant de 1 à 6, et de préférence égal à 6 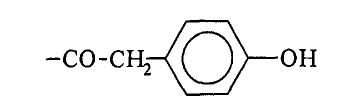 -SO₂-Flu
- CO-Flu
-CS-NH-Flu
Flu étant une molécule fluorescente
- H (correspondant à un nombre h)
- (CH₂)ₙH, n étant un nombre entier variant de 1 à 6
(correspondant à un nombre h)
- (CH₂)ₙ-OH n étant un nombre entier variant de 1 à 6
(correspondant à un nombre h)
- (CH₂)ₙ-SA' A' = H, CH₃ ou 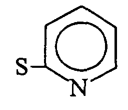 (correspondant à un nombre h) n étant un nombre entier variant de 1 à 6
avec
. i=u+j+k+h
. nombre total de α NH₃⁺ = p = u - q
. nombre total de ω NH₃⁺ = j = f - (k + h)
. nombre total de NH₃⁺ = m = p + j + 1
à condition que :
1) u ≥ i/2
2) m ≥ i/2

8. Conjugué oligomérique selon l'une quelconque des revendications 1 à 7, dans lequel le conjugué oligomérique contient un oligomère de formule selon la revendication 7, dans laquelle
i = 19 n = 4 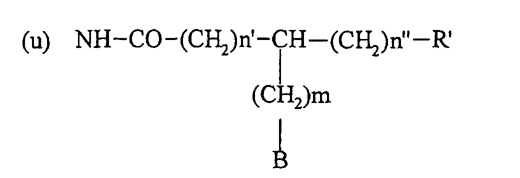 dans laquelle
n' = n"=0
R' = NH₃⁺
m = 1
B = imidazole
R = 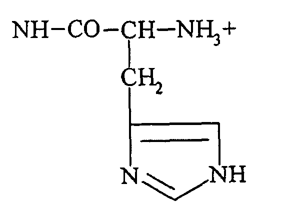
(f)R = NH⁺₃
u = 12
j = 7
ou
i = 19 n = 4 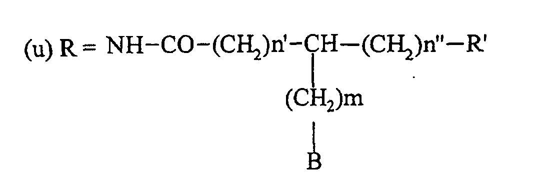 dans laquelle
n' = n" = 0
R' = NH₃⁺
m = 1
B = imidazole
R = 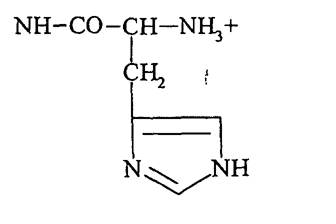
(f) R = NH₃⁺
u = 16
j = 3
ou
i = 19 n = 4 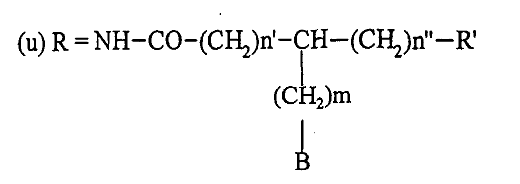 dans laquelle
n' = n" = 0
R' = NH₃⁺
m = 1
B = imidazole
R = 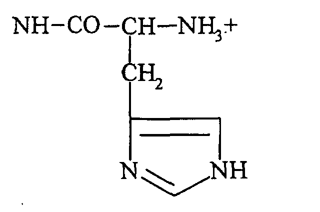
(f) R = NH₃⁺
u = 19
j = 0
ou
i = 19 n = 4 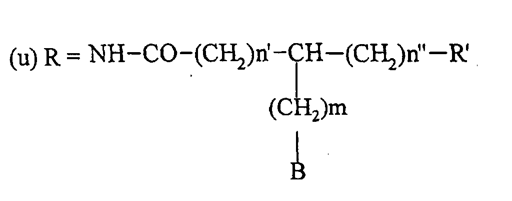 dans laquelle
n' = n" = 0
R' = NH₃⁺
m = 1
B = imidazole
R = 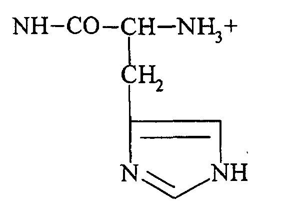
(f) R = CO-CH₃
u = 11
k = 8
i = 19 n = 4 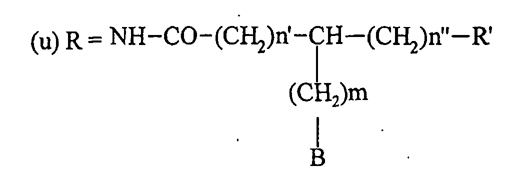 dans laquelle
n' = n" = 0
R' = NH₃⁺
m = 1
B = imidazole
R = 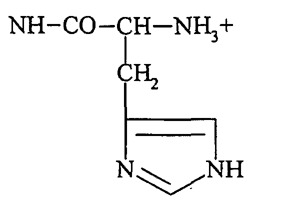
(f) R = CO-CH₃
u = 15
k = 4
ou
i = 19 n = 4 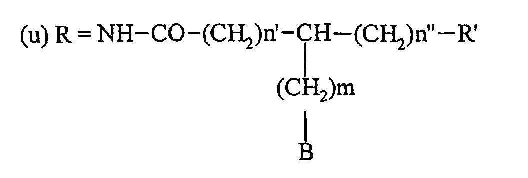 dans laquelle
n' = n" = 0
R' = NH₃⁺
m = 1
B = imidazole
R = 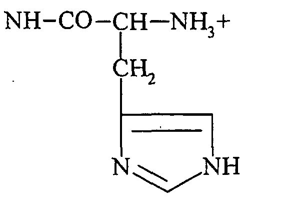
(f) R = CO-(CHOH)rH
r = 5
= 12
k = 3
ou
i = 19 n = 4 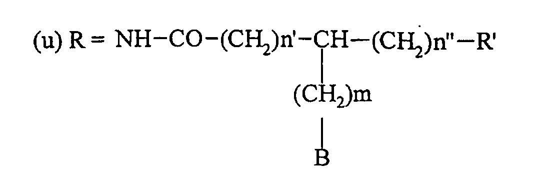 dans laquelle
(q) n' = n" = 20
R' = NH-CO-CH₃
m = 1
B = imidazole
R = 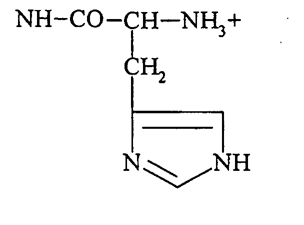
(f) R = NH₃⁺
u = 16
f = 4
k = 3

9. Composition contenant au moins un conjugué oligomérique selon l'une quelconque des revendications 1 à 8, en association avec au moins un peptide, un oligoside ou un oligonucléotide, ou un mélange de ceux-ci.

10. Préparation combinée contenant en tant que substance active les composants individuels suivants, sous la forme d'un kit of part :
- un conjugué oligomérique selon l'une quelconque des revendications 1 à 8,
- au moins un conjugué oligomérique selon l'une quelconque des revendications 1 à 8, en association avec au moins un peptide, un oligoside ou un oligonucléotide, ou un mélange de ceux-ci,
pour l'utilisation simultanée, séparée ou séquentielle, dans le cadre du transfert *in vitro, in vivo* ou *ex vivo* desdites molécules biologiques dans le cytosol et/ou le noyau cellulaire.

11. Utilisation d'un conjugué oligomérique selon l'une quelconque des revendications 1 à 8, pour la préparation d'un médicament destiné au transfert intracellulaire *in vitro, ex vivo* ou *in vivo* d'un peptide, d'un oligoside ou d'un oligonucléotide, dans le cytosol et/ou dans le noyau cellulaire.

12. Utilisation d'un conjugué oligomérique selon l'une quelconque des revendications 1 à 8, ou d'une composition selon la revendication 9, ou d'une préparation combinée selon la revendication 10, pour la préparation d'un médicament destiné au transfert intracellulaire *in vitro, ex vivo* ou *in vivo* d'un peptide, d'un oligoside ou d'un oligonucléotide, ou d'un mélange de ceux-ci, dans le cytosol et/ou dans le noyau cellulaire.

13. Utilisation selon la revendication 11 ou 12, dans laquelle les cellules sont choisies parmi les cellules musculaires, les cellules épithéliales, les cellules endothéliales, les cellules myéloïdes telles que les monocytes, les macrophages et les fibroblastes, les leucocytes et les granulocytes, les ostéoblastes ainsi que les cellules dendritiques, les cellules souches qui ne sont pas d'origine embryonnaire humaine, des cellules neuronales ou des cellules dermiques.

14. Procédé de transfert *in vitro* ou *ex vivo* d'un oligonucléotide, dans lequel un oligonucléotide et un conjugué oligomérique selon l'une quelconque des revendications 1 à 8 ou d'une composition selon la revendication 9, ou d'une préparation combinée selon la revendication 10, sont (est) mis en contact avec un milieu contenant les cellules à transférer, dans des conditions telles qu'il y a :
- transfert d'un oligonucléotide antisens dans le cytosol et/ou dans le noyau cellulaire où il se lie à et bloque la séquence d'ARNm complémentaire,
- ou transfert d'un oligonucléotide en tant qu'activateur dans le cytosol où il déprime ou active un second messager dans le cytosol, ou le gène correspondant dans le noyau,
- ou transfert dans le cytosol et/ou dans le noyau cellulaire d'oligonucléotides correspondant à une séquence d'ADN répétitive de type bactérien avec une activité stimulante ou immunodépressive,
- ou transfert d'un oligonucléotide dans le noyau cellulaire où il se lie à l'ADN et forme une triple hélice conduisant à l'inhibition de l'expression du gène,
- ou transfert dans le cytosol et/ou dans le noyau cellulaire de l'oligonucléotide ARN agissant comme leurres qui inhibent l'expression du gène en bloquant la liaison des facteurs de régulation avec la région d'ADN authentique,
- ou transfert dans le cytosol et/ou dans le noyau cellulaire de ribozymes (oligonucléotides ARN) qui inhibent l'expression du gène en clivant l'ARNm.

15. Procédé de transfert *in vitro* ou *ex vivo* de peptides, dans lequel un peptide ou un conjugué oligomérique selon l'une quelconque des revendications 1 à 8, ou une composition selon la revendication 9, ou une préparation combinée selon la revendication 10, notamment dans lequel le peptide est un peptide antigénique, sont (est) mis en contact avec un milieu contenant les cellules à transférer, dans des conditions telles qu'il y a un transfert dudit peptide antigénique dans le cytosol des cellules présentatrices d'antigènes (macrophages, cellules dendritiques et cellules B) dans lesquelles ils sont transformés en protéosomes afin de se lier aux molécules MHCI, permettant la présentation de l'épitope antigénique fixé sur MHCI.

16. Procédé de transfert *in vitro* ou *ex vivo* d'un oligoside, dans lequel un oligoside et un conjugué oligomérique selon l'une quelconque des revendications 1 à 8, ou une composition selon la revendication 9, ou une préparation combinée selon la revendication 10, sont (est) mis en contact avec un milieu contenant des cellules à transférer, dans des conditions telles qu'il y a transfert dudit oligoside dans le cytosol et/ou le noyau cellulaire.

17. Composition pharmaceutique comprenant, en tant que substance active, une composition selon la revendication 9, ou une préparation combinée selon la revendication 10, en association avec un véhicule pharmaceutiquement acceptable.

18. Utilisation d'une composition selon la revendication 9, ou d'une préparation combinée selon la revendication 10, pour la préparation d'un médicament pour une utilisation dans le cadre du traitement du cancer, de maladies inflammatoires ou immunologiques (telles que le rejet de greffe, les allergies, l'auto-immunité) ou de maladies infectieuses.

19. Kit ou trousse contenant :
- un conjugué oligomérique selon l'une quelconque des revendications 1 à 8, substitué par un résidu protonable conduisant, dans un milieu faiblement acide, dont le pH est inférieur à 7,4, à une déstabilisation des membranes cellulaires, ce conjugué oligomérique étant susceptible de comprendre un signal de reconnaissance, qui est fixé
ou non auparavant sur le susdit conjugué, ledit signal de reconnaissance étant dépendant de la cellule à cibler,
- au moins un peptide, un oligoside ou un oligonucléotide à transférer,
- éventuellement des réactifs permettant l'éventuelle liaison du signal de reconnaissance sur le susdit conjugué oligomérique,
- éventuellement des réactifs permettant la formation d'une composition selon la revendication 9, ou d'une préparation combinée selon la revendication 10,
- des réactifs permettant le transfert du peptide, de l'oligoside ou de l'oligonucléotide dans le cytosol et/ou le noyau cellulaire.
